(19) Europäisches Patentamt / European Patent Office / Office européen des brevets

(11) EP 1 918 000 A2

(12) EUROPEAN PATENT APPLICATION

(43) Date of publication:
**07.05.2008 Bulletin 2008/19**

(21) Application number: **08002889.7**

(22) Date of filing: **03.11.2004**

(51) Int Cl.:
***A61P 9/00*** (2006.01) ***A61P 3/00*** (2006.01)
***A61K 45/06*** (2006.01) ***A61K 31/397*** (2006.01)

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: **05.11.2003 US 517602 P**

(62) Document number(s) of the earlier application(s) in accordance with Art. 76 EPC:
**04800720.7 / 1 680 189**

(71) Applicant: **Schering Corporation**
**Kenilworth, NJ 07033-0530 (US)**

(72) Inventor: **Graziano, Michael P.**
**Scotch Plains**
**NJ 07076 (US)**

(74) Representative: **HOFFMANN EITLE**
**Patent- und Rechtsanwälte**
**Arabellastrasse 4**
**81925 München (DE)**

Remarks:
This application was filed on 15-02-2008 as a divisional application to the application mentioned under INID code 62.

# (54) Combinations of lipid modulating agents and substituted azetidinones and treatments for vascular conditions

(57) The present invention provides compositions, therapeutic combinations and methods including: (a) at least one lipid modulating agent; and (b) at least one substituted azetidinone or substituted -lactam sterol absorption inhibitor which can be useful for treating vascular conditions, diabetes, obesity and lowering plasma levels of sterols or 5 a-stanols.

EP 1 918 000 A2

**Description**

BACKGROUND OF THE INVENTION

FIELD OF THE INVENTION

The present invention relates to compositions and therapeutic combinations comprising certain lipid modulating agents and substituted azetidinones or substituted β-lactams for treating vascular and lipidemic conditions such as are associated with atherosclerosis, hypercholesterolemia and other vascular conditions in subjects.

Atherosclerotic coronary heart disease (CHD) represents the major cause for death and vascular morbidity in the western world. Risk factors for atherosclerotic coronary heart disease include hypertension, diabetes mellitus, family history, male gender, cigarette smoke and high serum cholesterol. A total cholesterol level in excess of 225-250 mg/dl is associated with significant elevation of risk of CHD. The newly revised NCEP ATP III low density lipoprotein (LDL-C) goal for patients with CHD or CHD risk equivalent is <100 mg/dL (2.59 mmol/L), for individuals with two or more risk factors is <130 mg/dL (3.37 mmol/L) and for individuals with fewer than two risk factors is <160 mg/dL (4.14 mmol/L).

The regulation of whole-body cholesterol homeostasis in mammals and animals involves the regulation of dietary cholesterol and modulation of cholesterol biosynthesis, bile acid biosynthesis and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis. When intestinal cholesterol absorption is reduced, by whatever means, less cholesterol is delivered to the liver. The consequence of this action is decreased hepatic lipoprotein (VLDL) production and an increase in the hepatic clearance of plasma cholesterol, mostly as LDL. Thus, the net effect of inhibiting intestinal cholesterol absorption is a decrease in plasma cholesterol levels and progression of atherosclerotic lesion formation.

U.S. Patents Nos. 5,767,115, 5,624,920, 5,668,990, 5,656,624 and 5,688,787, respectively, disclose hydroxy-substituted azetidinone compounds and substituted β-lactam compounds useful for lowering cholesterol and/or in inhibiting the formation of cholesterol-containing lesions in mammalian arterial walls. U.S. Patent No. 5,756,470, U.S. Patent Application No. 2002/0137690, U.S. Patent Application No. 2002/0137689 and PCT Patent Application No. WO 2002/066464 disclose sugar-substituted azetidinones and amino acid substituted azetidinones useful for preventing or treating atherosclerosis and reducing plasma cholesterol levels.

U.S. Patents Nos. 5,846,966 and 5,661,145, respectively, disclose treatments for inhibiting atherosclerosis and reducing plasma cholesterol levels using such hydroxy-substituted azetidinone compounds or substituted β-lactam compounds in combination with HMG CoA reductase inhibitor compounds, which act by blocking hydroxymethylglutaryl coenzyme A (HMG-CoA) reductase (the rate-limiting enzyme in hepatic cholesterol synthesis).

Despite recent improvements in the treatment of vascular disease, there remains a need for improved compounds, compositions and treatments for hyperlipidaemia, atherosclerosis and other vascular conditions that provide more efficient delivery of treatment.

SUMMARY OF THE INVENTION

In one embodiment, the present invention provides a composition comprising: (a) at least one lipid modulating agent; and (b) at least one substituted azetidinone compound or substituted β-lactam compound or pharmaceutically acceptable salt or solvate thereof.

In another embodiment, there is provided a composition comprising: (a) at least one lipid modulating agent; and (b) a compound represented by Formula (II) below:

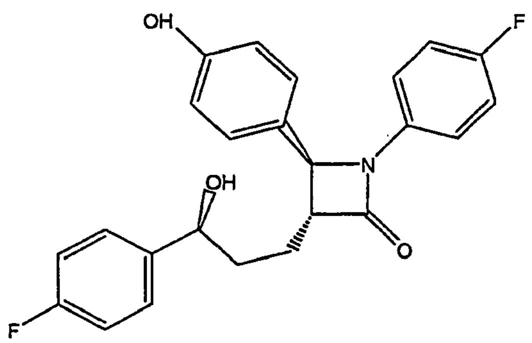

(II)

or pharmaceutically acceptable salt or solvate thereof.

Therapeutic combinations also are provided comprising: (a) a first amount of at least one lipid modulating agent; and (b) a second amount of at least one substituted azetidinone compound or substituted β-lactam compound or pharmaceutically acceptable salt or solvate thereof, wherein the first amount and the second amount together comprise a therapeutically effective amount for the treatment or prevention of a vascular condition, diabetes, obesity or lowering a concentration of a sterol in plasma of a subject.

Pharmaceutical compositions for the treatment or prevention of a vascular condition, diabetes, obesity or lowering a concentration of a sterol in plasma of a mammal, comprising a therapeutically effective amount of the above compositions or therapeutic combinations and a pharmaceutically acceptable carrier also are provided.

Methods of treating or preventing a vascular condition, diabetes, obesity or lowering a concentration of a sterol in plasma of a subject, comprising the step of administering to a mammal in need of such treatment an effective amount of the above compositions or therapeutic combinations also are provided.

Other than in the operating examples, or where otherwise indicated, all numbers expressing quantities of ingredients, reaction conditions, and so forth used in the specification and claims are to be understood as being modified in all instances by the term "about."

## DETAILED DESCRIPTION

The compositions and therapeutic combinations of the present invention comprise at least one "lipid modulating agent", which as used herein means a compound which functions as HDL, including synthetic HDL which contains lipid such as phosphotidyl choline, phosphatidyl serine, phosphatidyl ethanolamine, and other phospholipids in combination with HDL associated proteins such as apoA-I or variants thereof including apoAl-Milano and biologically active peptides derived therefrom, reverse lipid transport (RLT) peptides, enzymes associated with HDL such as paraoxonase, and apo E, alone or formulated in combination with liposomes or emulsions. See U.S Patent Application No. 2003/0109442 at pages 3-9, which is incorporated herein by reference. As used herein, HDL associated proteins include sequences present in HDL associated proteins that associate with HDL and synthetic peptides having equivalent binding or functional characteristics. Compounds which enhance HDL function include liposomes, where the HDL acts as a shuttle from the cells to the liposome. Suitable liposomal formulations are described in WO 95/23592 by the University of British Columbia.

The formulations described herein typically consist of an alpha helical protein such as an ApoA-I, a lipid, and a carrier.

Plasma ApoA-I is a single polypeptide chain of 243 amino acids, whose primary sequence is known (Brewer et al, Biochem. Biophys. Res. Commun. 80:623-630 (1978)). ApoA-I is synthesized as a 267 amino acid precursor in the cell. This preproapolipoproteinA-I is first intracellularly processed by N-terminal cleavage of 18 amino acids to yield proapolipoproteinA-I, and then further cleavage of 6 amino acids in the plasma or the lymph by the activity of specific proteases to yield apolipoproteinA-I. The major structural requirement of the ApoA-I molecule is believed to be the presence of repeat units of 11 or 22 amino acids, presumed to exist in amphipathic helical conformation (Segrest et al, FEBS Lett 38:247-253 (1974)). This structure allows for the main biological activities of ApoA-I, i.e. lipid binding and lecithin:cholesterol acyltransferase (LCAT) activation.

Human apolipoprotein Al-Milano (ApoA-IM) is a natural variant of ApoA-I (Weisgraber et al. J. Clin. Invest 66: 901-907 (1980)). In ApoA- IM the amino acid Arg173 is replaced by the amino acid Cys 173. Since ApoA-IM contains one Cys residue per polypeptide chain, it may exist in a monomeric, homodimeric, or heterodimeric form. These forms are chemically interchangeable, and the term ApoA-IM does not, in the present context, discriminate between these forms. On the DNA level the variant form results from a C to T substitution in the gene sequence, i.e. the codon CGC changed to TGC, allowing the translation of a cys instead of arg at amino acid position 173. However, this variant of ApoA-I is one of the most interesting variants, in that ApoA-IM subjects are characterized by a remarkable reduction in

HDL-cholesterol level, but without an apparent increased risk of arterial disease (Franceschini et al. J. Clin. Invest 66: 892-900 (1980)).

Another useful variant of ApoA-I is the Paris variant, where the arginine 151 is replaced with a cysteine.

The systemic infusion of ApoA-I alone (Miyazaki et al. Arterioscler Thromb Vasc Biol. 15:1882-1888(1995) or of HDL (Badimon et al, Lab Invest. 60:455-461 (1989) and J Clin Invest. 85:1234-1241 (1990)) in experimental animals and initial human clinical studies (Nanjee et al., Arterioscier Thromb Vasc Biol. 19:979- 989(1999) and Eriksson et al. Circulation. 100:594-598 (1999)) has been shown to exert significant biochemical changes, as well as to reduce the extent and severity of atherosclerotic lesions. It has now been discovered that it can be administered locally at a site of injury, and significantly reduce stenosis or restenosis, as discussed in more detail below and demonstrated by the following examples.

Other HDL-associated apolipoproteins with alphahelical characteristics could be used. Examples include Apo E, proApoA-I, ApoA- IParis, ApoA-II, proApoA-II, ApoA-IV, ApoC-I, ApoC-II, and ApoC-III, the alpha-helical sequences within these proteins, and apolipoproteins modified to include one or more sulfhydral groups, as described by Bielicki and Oda, Biochemistry 41:2089-2096 (2002). Additional HDL associated proteins can be used. Examples include paraoxonase, cholesteryl ester transfer protein, LCAT and phospholipid transfer protein. The above proteins can be used alone, in combination, complexed to lipid alone or in combination complexed to lipid. In addition, mixtures of complexes can be useful. An example is complexes comprised of ApoA-I with lipid and complexes comprised of paraoxanase with lipid administered as a mixture. Another example includes complexes comprised of greater than one protein component. For example, complexes comprised of ApoA-I, paraoxonase and lipid are useful.

Lipids form a complex with the ApoA-I which enhances its efficacy. Typically, the lipid is mixed with the ApoA-I prior to administration. Apolipoprotein and lipids are mixed in an aqueous solution in appropriate ratios and can be complexed by methods known in the art and including freeze-drying, detergent solubilization followed by dialysis, microfluidization, sonication, and homogenization. Complex efficiency can be optimized, for example, by varying pressure, ultrasonic frequency, or detergent concentration. An example of a detergent commonly used to prepared apolipoprotein-lipid complexes is sodium cholate.

In some cases it is desirable to mix the lipid and the apolipoprotein prior to administration. Lipids may be in solution or in the form of liposomes or emulsions formed using standard techniques such as sonication or extrusion. Sonication is generally performed with a tip sonifier, such as a Branson tip sonifier, in an ice bath. Typically, the suspension is subjected to several sonication cycles. Extrusion may be carried out by biomembrane extruders, such as the Lipex Biomembrane Extruder. Defined pore size in the extrusion filters may generate unilamellar liposomal vesicles of specific sizes. The liposomes may also be formed by extrusion through an asymmetric ceramic filter, such as a Ceraflow Microfilter, commercially available from the Norton Company, Worcester Mass. or through a polycarbonate filter or other types of polymerized materials (i.e. plastics) commonly known.

In some cases it is preferable to administer the apolipopotein alone, essentially lipid-free, to treat the injured artery. The aqueous sterile solution is added to the apolipoprotein. The apolipoprotein in solution can be administered to treat an injured artery. Alternative, freeze-dried preparation of complexes may be hydrated with an aqueous solution prior to administration. In other cases, frozen preparations of complexes in aqueous solution are thawed until a homogenous solution is achieved prior to administration to an injured vessel,

Preferred lipids are phospholipids, most preferably including at least one phospholipid, typically soy phosphatidylcholine, egg phosphatidylcholine, soy phosphatidylglycerol, egg phosphatidylglycerol, palmitoyl-oleoyl-phosphatidylcholine distearoylphosphatidylcholine, or distearoylphosphatidylglycerol. Other useful phospholipids include, e.g., phosphatidylcholine, phosphatidylglycerol, sphingomyelin, phosphatidylserine, phosphatidic acid, N-(2,3-di(9-(Z)-octadecenyloxy))- prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, stearoyl- palmitoyl-phosphatidylcholine, dipalmitoyl-phosphatidylethanolamine, distearoyl-phosphatidylethanolamine, dimyrstoyl-phosphatidylserine, and dioleyl-phosphatidylcholine. Non-phosphorus containing lipids may also be used, including stearylamine, docecylamine, acetyl palmitate, and fatty acid amides.

Additional lipids suitable for use are well known to persons of skill in the art and are cited in a variety of well known sources, e.g., McCutcheon's Detergents and Emulsifiers and McCutcheon's Functional Materials, Allured Publishing Co., Ridgewood, N.J., both of which are incorporated herein by reference. Generally, it is desirable that the lipids are liquid-crystalline at 37° C., 35° C., or 32° C. Lipids in the liquid-crystalline state typically accept cholesterol more efficiently than lipids in the gel state. As patients typically have a core temperature of about 37° C., lipids that are liquid-crystalline at 37° C. are generally in a liquid-crystalline state during treatment.

The concentration of the lipid in the formulation may vary. Persons of skill may vary these concentrations to optimize treatment with different lipid components or of particular patients. ApoAI is combined with lipid in a ratio by weight of between 1:0.5 to 1:3, with more lipid being preferred for clearance of cholesterol. A ratio of around 1:1 is preferred to produce the most homogenous population and for purposes of producing stable and reproducible batches.

In one embodiment, the lipid modulating agent is ETC-216, which is a synthetic HDL complex composed of 14 mg/mL of recombinant apolipoprotein A-I Milano and 13 mg/mL of 1-palmitoyl-2-oleoyl phosphatidyl choline (POPC) complex in sucrose-mannitol-phosphate buffer solution (sterile 6.4 % sucrose, 0.8% mannitol in 6 mmol/L phosphate buffer, pH 7.4) (Esperion Therapeutics, Inc.), as a ready to inject solution or saline.

In an alternative embodiment, genes encoding a protein to be delivered may be administered, rather than the protein. Gene transfer can be obtained using direct transfer of genetic material, in a plasmid or viral vector, or via transfer of genetic material in cells or carriers such as cationic liposomes. Such methods are well known in the art and readily adaptable for use in the gene mediated toxin therapies described herein. As reviewed by Francis, et al. Am. J. Pharmacogenomics 1(1):55-66 (2001), gene therapy offers a novel approach for prevention and treatment of cardiovascular diseases. Technical advances in viral vector systems and the development of fusigenic liposome vectors have been crucial to the development of effective gene therapy strategies directed at the vasculature and myocardium in animal models. Gene transfer techniques are being evaluated as potential treatment alternatives for both genetic (familial hypercholesterolemia) and acquired occlusive vascular diseases (atherosclerosis, restenosis, arterial thrombosis) as well as for cardiac disorders including heart failure, myocardial ischemia, graft coronary arteriosclerosis and hypertension. See also, Teiger, et al., J. Cardiovasc. Pharmacol. 33(5):726-732 (1999).

Studies by Wolff et al., Biotechniques 11:474-85 (1991), demonstrate injection of naked DNA into muscle allows long term and low expression levels of proteins coded for within the DNA sequence. Administration of naked DNA to smooth muscle layers can be achieved by use of an intramural device, such as an INFILTRATOR® and allow expression of the proteins or their alpha helical domains to treat the injured vessel. Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)). Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). As used herein, plasmid or viral vectors are agents that transport the gene into a cell without degradation and include a promoter yielding expression of the gene in the cell into which it is delivered. In a preferred embodiment vectors are derived from either a virus or a retrovirus. Preferred viral vectors are Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Preferred retroviruses include Murine Maloney Leukemia virus, MMLV, and retroviruses that express the desirable properties of MMLV as a vector.

Retroviral vectors are able to carry a larger genetic payload, i.e., a transgene or marker gene, than other viral vectors, and for this reason are a commonly used vector. However, they are not useful in non- proliferating cells. A retrovirus is an animal virus belonging to the virus family of Retroviridae, including any types, subfamilies, genus, or tropisms. Retroviral vectors, in general, are described by Verma, I. M., Retroviral vectors for gene transfer. In MICROBIOLOGY-1985, American Society for Microbiology, pp. 229-232, Washington, (1985), which is incorporated by reference herein. Examples of methods for using retroviral vectors for gene therapy are described in U.S. Pat. Nos. 4,868, 116 and 4,980,286; PCT applications WO 90/02806 and WO 89/07136; and Mulligan, (Science 260:926-932 (1993)).

Adenovirus vectors are relatively stable and easy to work with, have high titers, and can be delivered in aerosol formulation, and can transfect non-dividing cells. The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6: 75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287- 1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication- defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

Pox viral vectors are large and have several sites for inserting genes, they are thermostable and can be stored at room temperature. A preferred embodiment is a viral vector which has been engineered so as to suppress the immune

response of the host organism, elicited by the viral antigens. Preferred vectors of this type will carry coding regions for Interleukin 8 or 10.

Viral vectors have higher transaction (ability to introduce genes) abilities than do most chemical or physical methods to introduce genes into cells. Typically, viral vectors contain nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines that have been engineered to express the gene products of the early genes in trans.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273:113 (1978)). The immediate early promoter of the human cytomegalovirus is conveniently obtained as a HindIII E restriction fragment (Greenway, P. J. et al., Gene 18:355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78:993 (1981)) or 3' (Lusky, M. L., et al., Mol. Cell Bio. 3:1108 (1983)) to the transcription unit. Furthermore, enhancers can be within an intron (Banerji, J. L. et al., Cell 33:729 (1983)) as well as within the coding sequence itself (Osborne, T. F., et al., Mol. Cell Bio. 4:1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, $\alpha$-fetoprotein and insulin), typically one will use an enhancer from a eukaryotic cell virus. Preferred examples are the SV 40 enhancer on the late side of the replication origin (bp 100-270), the cytomegalovirus early promoter enhancer, the polyoma enhancer on the late side of the replication origin, and adenovirus enhancers.

The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

It is preferred that the promoter and/or enhancer region act as a constitutive promoter and/or enhancer to maximize expression of the region of the transcription unit to be transcribed. It is further preferred that the promoter and/or enhancer region be active in all eukaryotic cell types. A preferred promoter of this type is the CMV promoter (650 bases). Other preferred promoters are SV40 promoters, cytomegalovirus (full length promoter), and retroviral vector LTF. It has been shown that all specific regulatory elements can be cloned and used to construct expression vectors that are selectively expressed in specific cell types.

Expression vectors used in eukaryotic host cells may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In a preferred embodiment of the transcription unit, the polyadenylation region is derived from the SV40 early polyadenylation signal and consists of about 400 bases. It is also preferred that the transcribed units contain other standard sequences alone or in combination with the above sequences improve expression from, or stability of, the construct.

The viral vectors can include nucleic acid sequence encoding a marker product. This marker product is used to determine if the gene has been delivered to the cell and once delivered is being expressed. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure.

In a preferred embodiment, intramural delivery of DNA coding for ApoA-I, ApoA-IV, ApoE, paraoxonase or

alpha-helical regions within these proteins are delivered to an artery with or with out lipid to treat injured blood vessels.

DNA encoding a number of different proteins may also be delivered. For example, as described by Chen, et al., Jpn. J. Pharmacol. 89(4):327-336 (2002), cardiovascular gene transfer is not only a powerful technique for studying the function of specific genes in cardiovascular biology and pathobiology, but also a promising strategy for treating cardiovascular diseases. Since the mid-1990s, nitric oxide synthase (NOS), the enzyme that catalyzes the formation of nitric oxide (NO) from L- arginine, has received considerable attention as a potential candidate for cardiovascular gene therapy, because NO exerts critical and diverse functions in the cardiovascular system, and abnormalities in NO biology are apparent in a number of cardiovascular disease processes including cerebral vasospasm, atherosclerosis, postangioplasty restenosis, transplant vasculopathy, hypertension, diabetes mellitus, impotence and delayed wound healing. There are three NOS isoforms, i.e., endothelial (eNOS), neuronal (nNOS) and inducible (iNOS). All three NOS isoforms have been used in cardiovascular gene transfer studies with encouraging results.

Kipshidze, et al., J. Am. Coll. Cardio. 39(10):1686-1691 (2002) describes decreasing neointimal formation by intramural delivery of antisense oligonucleotides.

Turunen, et al., Mol Ther 6(3):306 (2002), describes gene therapy with nuclear targeted lacZ- and TIMP-1-encoding adenoviruses were coupled to a peptide-motif (HWGF) that can bind to matrix metalloproteinase (MMP)-2 and MMP-9. In vivo, local intravascular catheter-mediated gene transfer of a HWGF-targeted TIMP-1-encoding adenovirus (AdTIMP-1(HWGF)) significantly reduced intimal thickening in a rabbit aortic balloon denudation model compared with the control adenovirus.

This can provide for delivery and release over a much longer time period at the site in need of treatment.

The lipid modulating agent can be administered in a therapeutically effective amount and manner to treat the specified condition. In general, the formulation is administered at the site of treatment. The actual total dosage when delivered locally is significantly less than the dosage that would have to be administered systemically to achieve the same local dosage, however, the local concentration is much higher than the previous studies in which the ApoA- I was administered systemically. As noted above, the preferred dosages for ApoA-IM are between 4 and 6 mg ApoA-IM/vessel (typically up to three segments are treated with a total dosage of around 4 to 18 mg ApoA-IM), or between about 0.05 and 0.3 mg ApoA-IM/kg body weight in a 70 kg mammal. The preferred ratio of protein to lipid is between 1:0.5 to 1:3, with more lipid being preferred for clearance of cholesterol, but a more equal amount of protein to lipid being preferred for purposes of stability and consistency of preparations for regulatory approval. Ratios of protein to lipid for preparations other than those containing apoA-IM are tested at various ratios of protein to lipid and the stability and consistency, and characteristics (such as complex size and cholesterol efflux capacity) are determined for regulatory approval.

Although a single administration has been demonstrated to be efficacious, multiple dosages can be administered. For example, intravenous administration at day -1, 0, 1, 2 and 3 of 20 mg ApoA-IM/kg body weight resulted in all balloon over-stretched injured vessels showing increased lumen area relative to controls four weeks after the procedure.

The exact dose, however, is determined by the attending clinician and is dependent on such factors as the potency of the compound administered, the age, weight, condition and response of the patient.

The term "therapeutically effective amount" means that amount of therapeutic agents of the invention, such as the lipid modulating agent(s), substituted azetidinone(s) or substituted β-lactam(s) and other pharmacological or therapeutic agents described below, that will elicit a biological or medical response of a subject, tissue, system, animal or mammal that is being sought by the administrator (such as a researcher, doctor or veterinarian) which includes alleviation of the symptoms of the condition or disease being treated and the prevention, slowing or halting of progression of one or more conditions, for example vascular conditions, such as hyperlipidaemia (for example atherosclerosis, hypercholesterolemia or sitosterolemia), vascular inflammation, stroke, diabetes, obesity and/or to reduce the level of sterol(s) (such as cholesterol) in the plasma.

As used herein, "combination therapy" or "therapeutic combination" means the administration of two or more therapeutic agents, such as lipid modulating agent(s), substituted azetidinone(s) or substituted β-lactam(s), to prevent or treat a condition, for example a vascular condition, such as hyperlipidaemia (for example atherosclerosis, hypercholesterolemia or sitosterolemia), vascular inflammation, stroke, diabetes, obesity and/or reduce the level of sterol(s) (such as cholesterol) in the plasma or tissue. As used herein, "vascular" comprises cardiovascular, cerebrovascular and combinations thereof. The compositions, combinations and treatments of the present invention can be administered by any suitable means which produce contact of these compounds with the site of action in the body, for example in the plasma, liver or small intestine of a subject (mammal or human or other animal). Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating the condition. A potential advantage of the combination therapy disclosed herein may be a reduction in the required amount of an individual therapeutic compound or the overall total amount of therapeutic compounds that are effective in treating the condition. By using a combination of therapeutic agents, the side effects of the individual compounds can be reduced as compared to a monotherapy, which can improve patient

compliance. Also, therapeutic agents can be selected to provide a broader range of complimentary effects or complimentary modes of action.

As discussed above, the compositions, pharmaceutical compositions and therapeutic combinations of the present invention comprise one or more substituted azetidinone or substituted β-lactam sterol absorption inhibitors discussed in detail below. As used herein, "sterol absorption inhibitor" means a compound capable of inhibiting the absorption of one or more sterols, including but not limited to cholesterol, phytosterols (such as sitosterol, campesterol, stigmasterol and avenosterol), 5α-stanols (such as cholestanol, 5α-campestanol, 5α-sitostanol), and/or mixtures thereof, when administered in a therapeutically effective (sterol and/or 5α-stanol absorption inhibiting) amount to a mammal or human.

In one embodiment, substituted azetidinones useful in the compositions, therapeutic combinations and methods of the present invention are represented by Formula (I) below:

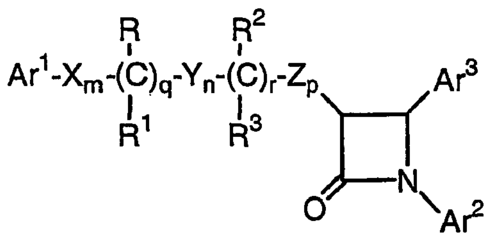

(I)

or pharmaceutically acceptable salts or solvates of the compounds of Formula (I), wherein, in Formula (I) above:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1; r is 0 or 1; m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$,
$-NR^6(CO)OR^9$, $-NR^6(CO)NR^7R^8$, $-NR^6SO_2R^9$, $-COOR^6$,$-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

Preferably, $R^4$ is 1-3 independently selected substituents, and $R^5$ is preferably 1-3 independently selected substituents.

As used herein, the term "alkyl" or "lower alkyl" means straight or branched alkyl chains having from 1 to 6 carbon atoms and "alkoxy" means alkoxy groups having 1 to 6 carbon atoms. Non-limiting examples of lower alkyl groups include, for example methyl, ethyl, propyl, and butyl groups.

"Alkenyl" means straight or branched carbon chains having one or more double bonds in the chain, conjugated or unconjugated. Similarly, "alkynyl" means straight or branched carbon chains having one or more triple bonds in the chain. Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Aryl" means phenyl, naphthyl, indenyl, tetrahydronaphthyl or indanyl.

"Phenylene" means a bivalent phenyl group, including ortho, meta and para-substitution.

The statements wherein, for example, R, $R^1$, $R^2$ and $R^3$, are said to be independently selected from a group of substituents, mean that R, $R^1$, $R^2$ and $R^3$ are independently selected, but also that where an R, $R^1$, $R^2$ and $R^3$ variable occurs more than once in a molecule, each occurrence is independently selected (e.g., if R is $-OR^6$, wherein $R^6$ is hydrogen, $R^2$ can be $-OR^6$ wherein $R^6$ is lower alkyl). Those skilled in the art will recognize that the size and nature of the substituent(s) will affect the number of substituents that can be present.

Compounds of the invention have at least one asymmetrical carbon atom and therefore all isomers, including enantiomers, stereoisomers, rotamers, tautomers and racemates of the compounds of Formula (I-XI) (where they exist) are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of the Formulae I-XI. Isomers may also include geometric isomers, e.g., when a double bond is present.

Those skilled in the art will appreciate that for some of the compounds of the Formulae I-XI, one isomer will show greater pharmacological activity than other isomers.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

As used herein, "solvate" means a molecular or ionic complex of molecules or ions of solvent with those of solute (for example, one or more compounds of Formulae I-XI, isomers of the compounds of Formulae I-XI, or prodrugs of the compounds of Formulae I-XI). Non-limiting examples of useful solvents include polar, protic solvents such as water and/or alcohols (for example methanol).

As used herein, "prodrug" means compounds that are drug precursors which, following administration to a patient, release the drug *in vivo* via some chemical or physiological process (e.g., a prodrug on being brought to the physiological pH or through enzyme action is converted to the desired drug form).

Preferred compounds of Formula (I) are those in which $Ar^1$ is phenyl or $R^4$-substituted phenyl, more preferably $(4-R^4)$-substituted phenyl. $Ar^2$ is preferably phenyl or $R^4$-substituted phenyl, more preferably $(4-R^4)$-substituted phenyl. $Ar^3$ is preferably $R^5$-substituted phenyl, more preferably $(4-R^5)$-substituted phenyl. When $Ar^1$ is $(4-R^4)$-substituted phenyl, $R^4$ is preferably a halogen. When $Ar^2$ and $Ar^3$ are $R^4$- and $R^5$-substituted phenyl, respectively, $R^4$ is preferably halogen or $-OR^6$ and $R^5$ is preferably $-OR^6$, wherein $R^6$ is lower alkyl or hydrogen. Especially preferred are compounds wherein each of $Ar^1$ and $Ar^2$ is 4-fluorophenyl and $Ar^3$ is 4-hydroxyphenyl or 4-methoxyphenyl.

X, Y and Z are each preferably $-CH_2-$. $R^1$ and $R^3$ are each preferably hydrogen. R and $R^2$ are preferably $-OR^6$ wherein $R^6$ is hydrogen, or a group readily metabolizable to a hydroxyl (such as $-O(CO)R^6$, $-O(CO)OR^9$ and $-O(CO)NR^6R^7$, defined above).

The sum of m, n, p, q and r is preferably 2, 3 or 4, more preferably 3. Preferred are compounds wherein m, n and r are each zero, q is 1 and p is 2.

Also preferred are compounds of Formula (I) in which p, q and n are each zero, r is 1 and m is 2 or 3. More preferred are compounds wherein m, n and r are each zero, q is 1, p is 2, Z is $-CH_2$ and R is $-OR^6$, especially when $R^6$ is hydrogen.

Also more preferred are compounds of Formula (I) wherein p, q and n are each zero, r is 1, m is 2, X is $-CH_2-$ and $R^2$ is $-OR^6$, especially when $R^6$ is hydrogen.

Another group of preferred compounds of Formula (I) is that in which $Ar^1$ is phenyl or $R^4$-substituted phenyl, $Ar^2$ is phenyl or $R^4$-substituted phenyl and $Ar^3$ is $R^5$-substituted phenyl. Also preferred are compounds in which $Ar^1$ is phenyl or $R^4$-substituted phenyl, $Ar^2$ is phenyl or $R^4$-substituted phenyl, $Ar^3$ is $R^5$-substituted phenyl, and the sum of m, n, p, q and r is 2, 3 or 4, more preferably 3.

More preferred are compounds wherein $Ar^1$ is phenyl or $R^4$-substituted phenyl, $Ar^2$ is phenyl or $R^4$-substituted phenyl, $Ar^3$ is $R^5$-substituted phenyl, and wherein m, n and r are each zero, q is 1 and p is 2, or wherein p, q and n are each zero, r is 1 and m is 2 or 3.

In a preferred embodiment, a substituted azetidinone of Formula (I) useful in the compositions, therapeutic combinations and methods of the present invention is represented by Formula (II) (ezetimibe) below:

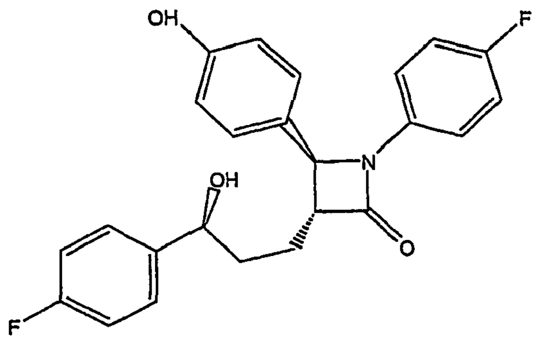

(II)

or pharmaceutically acceptable salts or solvates of the compound of Formula (II).
The compound of Formula (II) can be in anhydrous or hydrated form. A product containing ezetimibe compound is commercially available as ZETIA® ezetimibe formulation from MSP Pharmaceuticals.

Compounds of Formula I can be prepared by a variety of methods well know to those skilled in the art, for example such as are disclosed in U.S. Patents Nos. 5,631,365, 5,767,115, 5,846,966, 6,207,822, PCT Patent Application No. 02/079174, and PCT Patent Application WO 93/02048, each of which is incorporated herein by reference, and in the Example below.

Alternative substituted azetidinones useful in the compositions, therapeutic combinations and methods of the present invention are represented by Formula (III) below:

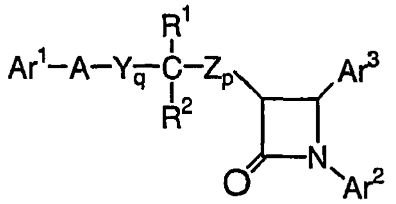

(III)

or a pharmaceutically acceptable salt thereof or a solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O- -S-, -S(O)- or $-S(O)_2$ ;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl, $-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;
$R^3$ and $R^4$ are independently 1-3 substituents independently selected from the group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and $R^9$ is lower alkyl, aryl or aryl-substituted lower alkyl.

Methods for making compounds of Formula III are well known to those skilled in the art. Non-limiting examples of suitable methods are disclosed in U.S. Patent No. 5,688,990, which is incorporated herein by reference.

In another embodiment, substituted azetidinones useful in the compositions, therapeutic combinations and methods of the present invention are represented by Formula (IV):

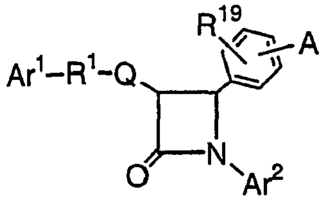

(IV)

or a pharmaceutically acceptable salt thereof or a solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

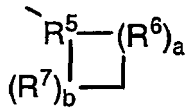

;

and
$R^1$ is selected from the group consisting of:

$-(CH_2)_q-$, wherein q is 2-6, provided that when Q forms a spiro ring, q can also be zero or 1;
$-(CH_2)_e-G-(CH_2)_r-$, wherein G is -O-, -C(O)-, phenylene, $-NR^8-$ or $-S(O)_{0-2}-$, e is 0-5 and r is 0-5, provided that the sum of e and r is 1-6;
$-(C_2-C_6$ alkenylene)-; and
$-(CH_2)_f V-(CH_2)_g-$, wherein V is $C_3-C_6$ cycloalkylene, f is 1-5 and g is 0-5, provided that the sum of f and g is 1-6;

$R^5$ is selected from:

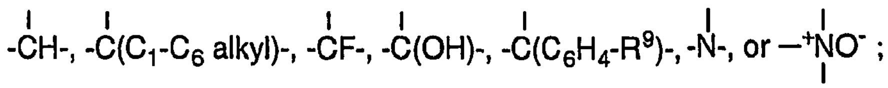

$R^6$ and $R^7$ are independently selected from the group consisting of $-CH_2-$, $-CH(C_1-C_6$ alkyl)-, $-C(di-(C_1-C_6)$ alkyl), -CH=CH- and- $C(C_1-C_6$ alkyl)=CH-; or $R^5$ together with an adjacent $R^6$, or $R^5$ together with an adjacent $R^7$, form a -CH=CH- or a $-CH=C(C_1-C_6$ alkyl)- group;
a and b are independently 0, 1, 2 or 3, provided both are not zero; provided that when $R^6$ is -CH=CH- or $-C(C_1-C_6$ alkyl)=CH-, a is 1; provided that when $R^7$ is
-CH=CH- or $-C(C_1-C_6$ alkyl)=CH-, b is 1; provided that when a is 2 or 3, the $R^6$'s can be the same or different; and provided that when b is 2 or 3, the $R^7$'s can be the same or different;
and when Q is a bond, $R^1$ also can be selected from:

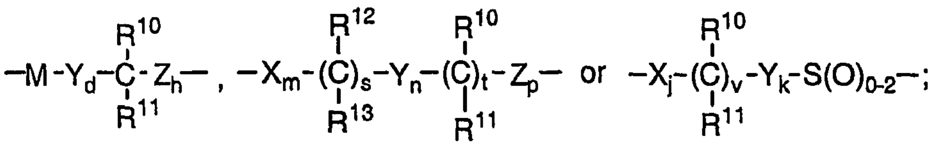

where M is -O-, -S-, -S(O)- or $-S(O)_2$ ;
X, Y and Z are independently selected from the group consisting of $-CH_2-$, $-CH(C_1-C_6$ alkyl)- and $-C(di-(C_1-C_6)$ alkyl);
$R^{10}$ and $R^{12}$ are independently selected from the group consisting of $-OR^{14}$, $-O(CO)R^{14}$, $-O(CO)OR^{16}$ and $-O(CO)NR^{14}R^{15}$;
$R^{11}$ and $R^{13}$ are independently selected from the group consisting of hydrogen, $(C_1-C_6)$alkyl and aryl; or $R^{10}$ and $R^{11}$ together are =O, or $R^{12}$ and $R^{13}$ together are =O;
d is 1,2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, $(C_1-C_{10})$alkyl, $(C_2-C_{10})$alkenyl, $(C_2-C_{10})$alkynyl, $(C_3-C_6)$cycloalkyl, $(C_3-C_6)$cycloalkenyl, $R^{17}$-substituted aryl, $R^{17}$-substituted benzyl,
$R^{17}$-substituted benzyloxy, $R^{17}$-substituted aryloxy, halogeno, $-NR^{14}R^{15}$, $NR^{14}R^{15}(C_1-C_6$ alkylene)-, $NR^{14}R^{15}C(O)(C_1-C_6$ alkylene)-,-NHC(O)$R^{16}$, OH, $C_1-C_6$ alkoxy, $-OC(O)R^{16}$,
$-COR^{14}$, hydroxy$(C_1-C_6)$alkyl, $(C_1-C_6)$alkoxy$(C_1-C_6)$alkyl, $NO_2$, $-S(O)_{0-2}R^{16}$,$-SO_2NR^{14}R^{15}$ and $-(C_1-C_6$ alkylene)$COOR^{14}$; when $R^2$ is a substituent on a heterocycloalkyl ring, $R^2$ is as defined, or is =O or

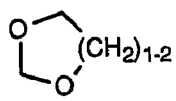 ;

and, where $R^2$ is a substituent on a substitutable ring nitrogen, it is hydrogen, $(C_1-C_6)$alkyl, aryl, $(C_1-C_6)$alkoxy, aryloxy, $(C_1-C_6)$alkylcarbonyl, arylcarbonyl, hydroxy, $-(CH_2)_{1-6}CON^{18}R^{18}$,

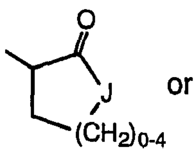 or 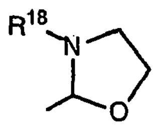 ;

wherein J is -O-, -NH-, $-NR^{18}-$ or $-CH_2-$;
$R^3$ and $R^4$ are independently selected from the group consisting of 1-3 substituents independently selected from the group consisting of $(C_1-C_6)$alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}COOR^{14}$, $-O(CH_2)_{1-10}CONR^{14}R^{15}$, $-(C_1-C_6$ alkylene)-$COOR^{14}$, -CH=CH-$COOR^{14}$, $-CF_3$, -CN, $-NO_2$ and halogen;
$R^8$ is hydrogen, $(C_1-C_6)$alkyl, aryl $(C_1-C_6)$alkyl, $-C(O)R^{14}$ or $-COOR^{14}$;
$R^9$ and $R^{17}$ are independently 1-3 groups independently selected from the group consisting of hydrogen, $(C_1-C_6)$alkyl, $(C_1-C_6)$alkoxy, -COOH, $NO_2$, $-NR^{14}R^{15}$, OH and halogeno;
$R^{14}$ and $R^{15}$ are independently selected from the group consisting of hydrogen, $(C_1-C_6)$alkyl, aryl and aryl-substituted $(C_1-C_6)$alkyl;
$R^{16}$ is $(C_1-C_6)$alkyl, aryl or $R^{17}$-substituted aryl;
$R^{18}$ is hydrogen or $(C_1-C_6)$alkyl; and
$R^{19}$ is hydrogen, hydroxy or $(C_1-C_6)$alkoxy.

Methods for making compounds of Formula IV are well known to those skilled in the art. Non-limiting examples of suitable methods are disclosed in U.S. Patent No. 5,656,624, which is incorporated herein by reference.

In another embodiment, substituted azetidinones useful in the compositions, therapeutic combinations and methods of the present invention are represented by Formula (V):

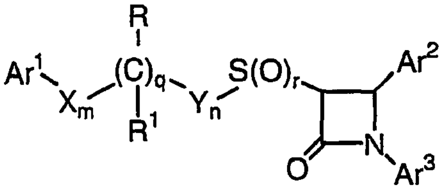

(V)

or a pharmaceutically acceptable salt thereof or a solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;
q is 0 or 1;
r is 0, 1 or 2;
m and n are independently 0, 1, 2, 3, 4 or 5; provided that the sum of m, n and q is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $- NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $- SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $- NO_2$, halogen, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen.

Methods for making compounds of Formula V are well known to those skilled in the art. Non-limiting examples of suitable methods are disclosed in U.S. Patent No. 5,624,920, which is incorporated herein by reference.

In another embodiment, substituted azetidinones useful in the compositions, therapeutic combinations and methods of the present invention are represented by Formula (VI):

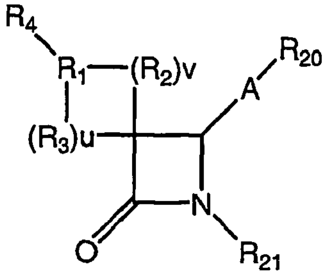

(VI)

or a pharmaceutically acceptable salt thereof or a solvate thereof, wherein:

$R_1$ is

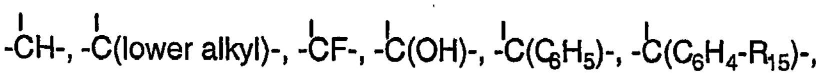

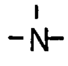 or 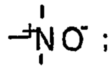 ;

$R_2$ and $R_3$ are independently selected from the group consisting of: $-CH_2-$, -CH(lower alkyl)-, -C(di-lower alkyl)-, -CH=CH- and -C(lower alkyl)=CH-; or

$R_1$ together with an adjacent $R_2$, or $R_1$ together with an adjacent $R_3$, form a -CH=CH- or a -CH=C(lower alkyl)- group;

u and v are independently 0, 1, 2 or 3, provided both are not zero; provided that when $R_2$ is -CH=CH- or -C(lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;

$R_4$ is selected from $B-(CH_2)_mC(O)-$, wherein m is 0, 1, 2, 3, 4 or 5; $B-(CH_2)_q-$, wherein q is 0, 1, 2, 3, 4, 5 or 6; $B-(CH_2)_e-Z-(CH_2)_r-$, wherein Z is -O-,

-C(O)-, phenylene, $-N(R_8)-$ or $-S(O)_{0-2}-$, e is 0, 1, 2, 3, 4 or 5 and r is 0, 1, 2, 3, 4 or 5, provided that the sum of e and r is 0, 1, 2, 3, 4, 5 or 6; B-($C_2$-$C_6$ alkenylene)-; B-($C_4$-$C_6$ alkadienylene)-; $B-(CH_2)_t-Z-$($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6; $B-(CH_2)_f-V-(CH_2)_g-$, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6; $B-(CH_2)_t-V-$($C_2$-$C_6$ alkenylene)- or B-($C_2$-$C_6$ alkenylene)-$V-(CH_2)_t-$, wherein V and t are as defined above, provided that the sum of t and the number of carbon atoms in the alkenylene chain is 2, 3, 4, 5 or 6;

$B-(CH_2)_a-Z-(CH_2)_b-V-(CH_2)_d-$, wherein Z and V are as defined above and a, b and d are independently 0,1, 2, 3, 4, 5 or 6, provided that the sum of a, b and d is 0, 1, 2, 3, 4, 5 or 6; or $T-(CH_2)_s-$, wherein T is cycloalkyl of 3-6 carbon atoms and s is 0, 1, 2, 3, 4, 5 or 6; or

$R_1$ and $R_4$ together form the group

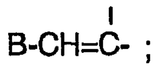

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

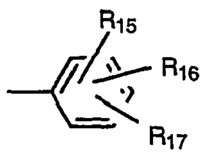

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl, $R_7$-benzyl, benzyloxy, $R_7$-benzyloxy, phenoxy, $R_7$-phenoxy, dioxolanyl, $NO_2$,$-N(R_8)(R_9)$, $N(R_8)(R_9)$-lower alkylene-, $N(R_8)(R_9)$-lower alkylenyloxy-, OH, halogeno, -CN, $-N_3$, $-NHC(O)OR_{10}$,

$-NHC(O)R_{10}$, $R_{11}O_2SNH-$, $(R_{11}O_2S)_2N-$, $-S(O)_2NH_2$, $-S(O)_{0-2}R_8$, tert-butyldimethyl-silyloxymethyl, $-C(O)R_{12}$, $-COOR_{19}$, $-CON(R_8)(R_9)$,$-CH=CHC(O)R_{12}$,- lower alkylene-$C(O)R_{12}$, $R_{10}C(O)$(lower alkylenyloxy)-, $N(R_8)(R_9)C(O)$(lower alkylenyloxy)- and

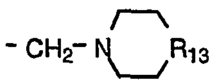

for substitution on ring carbon atoms,

and the substituents on the substituted heteroaryl ring nitrogen atoms, when present, are selected from the group consisting of lower alkyl, lower alkoxy, - C(O)OR$_{10}$, -C(O)R$_{10}$, OH, N(R$_8$)(R$_9$)-lower alkylene-, N(R$_8$)(R$_9$)-lower alkylenyloxy-, -S(O)$_2$NH$_2$ and 2-(trimethylsilyl)-ethoxymethyl;

R$_7$ is 1-3 groups independently selected from the group consisting of lower alkyl, lower alkoxy, -COOH, NO$_2$, -N (R$_8$)(R$_9$), OH, and halogeno;

R$_8$ and R$_9$ are independently selected from H or lower alkyl;

R$_{10}$ is selected from lower alkyl, phenyl, R$_7$-phenyl, benzyl or R$_7$-benzyl;

R$_{11}$ is selected from OH, lower alkyl, phenyl, benzyl, R$_7$-phenyl or R$_7$-benzyl;

R$_{12}$ is selected from H, OH, alkoxy, phenoxy, benzyloxy,

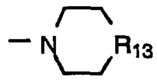

,

-N(R$_8$)(R$_9$), lower alkyl, phenyl or R$_7$-phenyl;

R$_{13}$ is selected from -O-, -CH$_2$-, -NH-, -N(lower alkyl)- or -NC(O)R$_{19}$;

R$_{15}$, R$_{16}$ and R$_{17}$ are independently selected from the group consisting of H and the groups defined for W; or R$_{15}$ is hydrogen and R$_{16}$ and R$_{17}$, together with adjacent carbon atoms to which they are attached, form a dioxolanyl ring;

R$_{19}$ is H, lower alkyl, phenyl or phenyl lower alkyl; and

R$_{20}$ and R$_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

Methods for making compounds of Formula VI are well known to those skilled in the art. Non-limiting examples of suitable methods are disclosed in U.S. Patent No. 5,698,548, which is incorporated herein by reference.

In another embodiment, substituted azetidinones useful in the compositions, therapeutic combinations and methods of the present invention are represented by Formulas (VIIA) and (VIIB):

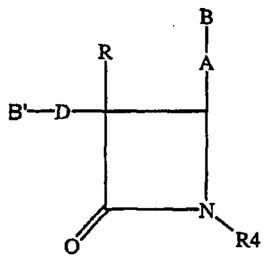

(VIIA)

and

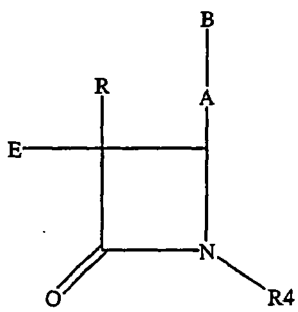

(VIIB)

or a pharmaceutically acceptable salt or solvate thereof, wherein:

A is -CH=CH-, -C≡C- or $-(CH_2)_p-$ wherein p is 0, 1 or 2;
B is

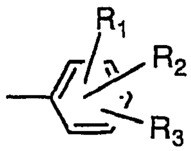

B'is

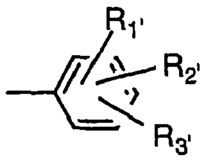

D is $-(CH_2)_mC(O)-$ or $-(CH_2)_q-$ wherein m is 1, 2, 3 or 4 and q is 2, 3 or 4;
E is $C_{10}$ to $C_{20}$ alkyl or -C(O)-($C_9$ to $C_{19}$)-alkyl, wherein the alkyl is straight or branched, saturated or containing one or more double bonds;
R is hydrogen, $C_1$-$C_{15}$ alkyl, straight or branched, saturated or containing one or more double bonds, or B-$(CH_2)_r$ -, wherein r is 0, 1, 2, or 3;
$R_1$, $R_2$, $R_3$, $R_{1'}$, $R_{2'}$, and $R_{3'}$ are independently selected from the group consisting of hydrogen, lower alkyl, lower alkoxy, carboxy, $NO_2$, $NH_2$, OH, halogeno, lower alkylamino, dilower alkylamino, -NHC(O)$OR_5$, $R_6O_2$SNH- and - S $(O)_2$NH2;
$R_4$ is

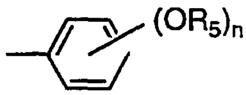

wherein n is 0, 1, 2 or 3;
$R_5$ is lower alkyl; and
$R_6$ is OH, lower alkyl, phenyl, benzyl or substituted phenyl wherein the substituents are 1-3 groups independently selected from the group consisting of lower alkyl, lower alkoxy, carboxy, $NO_2$, $NH_2$, OH, halogeno, lower alkylamino and dilower alkylamino; or a pharmaceutically acceptable salt thereof or a solvate thereof.

In another embodiment, sterol absorption inhibitors useful in the compositions and methods of the present invention are represented by Formula (VIII):

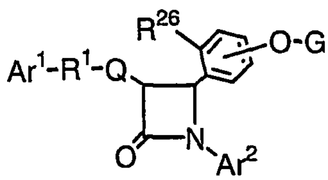
(VIII)

or a pharmaceutically acceptable salt thereof or a solvate thereof, wherein, in Formula (VIII) above,
$R^{26}$ is H or $OG^1$;
G and $G^1$ are independently selected from the group consisting of H,

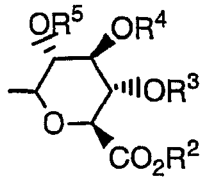 , 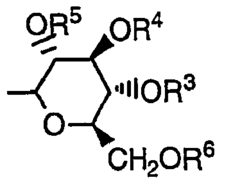 , 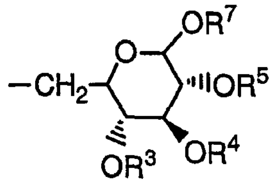

and

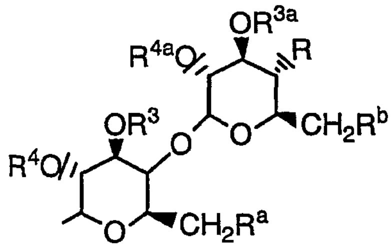 ;

provided that when $R^{26}$ is H or OH, G is not H;

R, $R^a$ and $R^b$ are independently selected from the group consisting of H, - OH, halogeno, $-NH_2$, azido, $(C_1-C_6)$alkoxy $(C_1-C_6)$-alkoxy or $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, R3a and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is selected from the group consisting of $R^{32}$-substituted T, $R^{32}$-substituted-T-$(C_1-C_6)$alkyl, $R^{32}$-substituted-$(C_2-C_4)$ alkenyl, $R^{32}$-substituted-$(C_1-C_6)$alkyl, $R^{32}$-substituted-$(C_3-C_7)$cycloalkyl and $R^{32}$-substituted-$(C_3-C_7)$cycloalkyl$(C_1-C_6)$ alkyl;

$R^{31}$ is selected from the group consisting of H and $(C_1-C_4)$alkyl;

T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsuffinyl, $(C_1-C_4)$alkylsulfonyl, $-N(CH_3)_2$, $-C(O)-NH(C_1-C_4)$alkyl, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

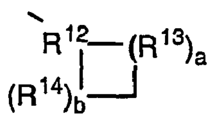 ;

and

$R^1$ is selected from the group consisting of

$-(CH_2)q-$, wherein q is 2-6, provided that when Q forms a spiro ring, q can also be zero or 1;

$-(CH_2)_e-E-(CH_2)_r-$, wherein E is -O-, -C(O)-, phenylene, -NR22- or $-S(O)_{0-2}-$, e is 0-5 and r is 0-5, provided that the sum of e and r is 1-6;

$-(C_2-C_6)$alkenylene-; and

$-(CH_2)_f-V-(CH_2)g-$, wherein V is $C_3-C_6$ cycloalkylene, f is 1-5 and g is 0-5, provided that the sum of f and g is 1-6;

$R^{12}$ is

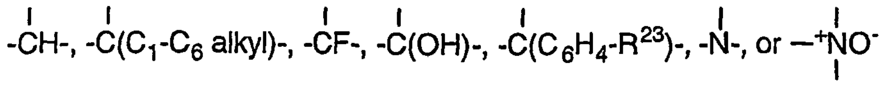 ;

$R^{13}$ and $R^{14}$ are independently selected from the group consisting of $-CH_2-$, $-CH(C_1-C_6$ alkyl$)-$, $-C($di$-(C_1-C_6)$ alkyl$)$, $-CH=CH-$ and $-C(C_1-C_6$ alkyl$)=CH-$; or $R^{12}$ together with an adjacent $R^{13}$, or $R^{12}$ together with an adjacent $R^{14}$, form a $-CH=CH-$ or a $-CH=C(C_1-C_6$ alkyl$)-$ group;

a and b are independently 0, 1, 2 or 3, provided both are not zero;

provided that when $R^{13}$ is $-CH=CH-$ or $-C(C_1-C_6$ alkyl$)=CH-$, a is 1;

provided that when $R^{14}$ is $-CH=CH-$ or $-C(C_1-C_6$ alkyl$)=CH-$, b is 1;

provided that when a is 2 or 3, the $R^{13}$'s can be the same or different; and

provided that when b is 2 or 3, the $R^{14}$'s can be the same or different;

and when Q is a bond, $R^1$ also can be:

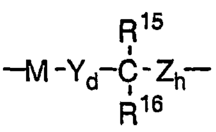 , 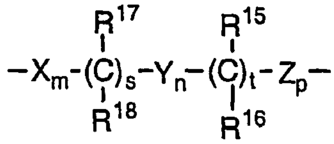 or 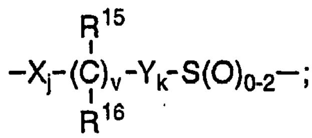 ;

M is $-O-$, $-S-$, $-S(O)-$ or $-S(O)_2-$;

X, Y and Z are independently selected from the group consisting of $-CH_2-$, $-CH(C_1-C_6)$alkyl- and $-C($di$-(C_1-C_6)$alkyl$)$;

$R^{10}$ and $R^{11}$ are independently selected from the group consisting of 1-3 substituents independently selected from the group consisting of $(C_1-C_6)$alkyl, $-OR^{19}$, $-O(CO)R^{19}$, -O(CO)OR21, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, -COOR19, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene$)-COOR^{19}$, $-CH=CH-COOR^{19}$, $-CF_3$, -CN, $-NO_2$ and halogen;

$R^{15}$ and $R^{17}$ are independently selected from the group consisting of $-OR^{19}$, $-O(CO)R^{19}$, $-O(CO)OR^{21}$ and $-O(CO)NR^{19}R^{20}$;

$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;

d is 1, 2 or 3;

h is 0, 1, 2, 3 or 4;

s is 0 or 1; t is 0 or 1; m, n and p are independently 0-4;

provided that at least one of s and t is 1, and the sum of m, n, p, s and t is 1-6;

provided that when p is 0 and t is 1, the sum of m, s and n is 1-5; and provided that when p is 0 and s is 1, the sum of m, t and n is 1-5;

v is 0 or 1;

j and k are independently 1-5, provided that the sum of j, k and v is 1-5;

and when Q is a bond and $R^1$ is

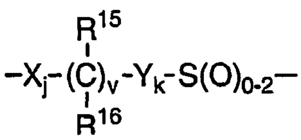 ,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, $(C_1-C_6)$alkyl, aryl and aryl-substituted $(C_1-C_6)$alkyl;

R21 is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or$-COOR^{19}$;

R23 and $R^{24}$ are independently 1-3 groups independently selected from the group consisting of H, $(C_1\text{-}C_6)$alkyl, $(C_1\text{-}C_6)$ alkoxy, -COOH, $NO_2$ -$NR^{19}R^{20}$, -OH and halogeno; and
$R^{25}$ is H, -OH or $(C_1\text{-}C_6)$alkoxy.

Methods for making compounds of Formula VIII are well known to those skilled in the art. Non-limiting examples of suitable methods are disclosed in U.S. Patent No. 5,756,470, which is incorporated herein by reference.

In another embodiment, substituted azetidinones useful in the compositions and methods of the present invention are represented by Formula (IX) below:

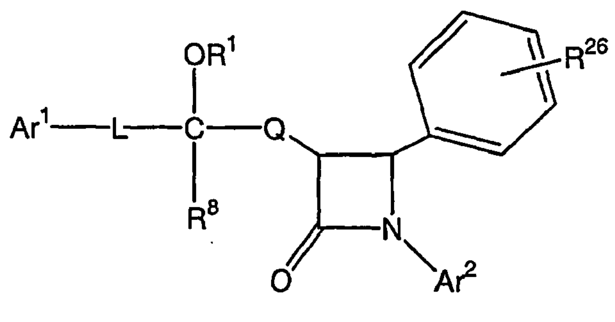

(IX)

or a pharmaceutically acceptable salt or solvate thereof, wherein in Formula (IX):

$R^1$ is selected from the group consisting of H, G, $G^1$, $G^2$, -$SO_3H$ and - $PO_3H$;
G is selected from the group consisting of: H,

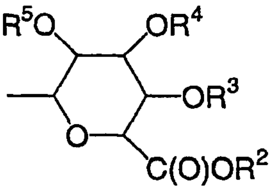 , 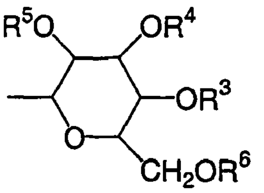 , 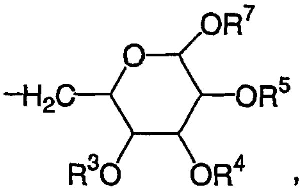 ,

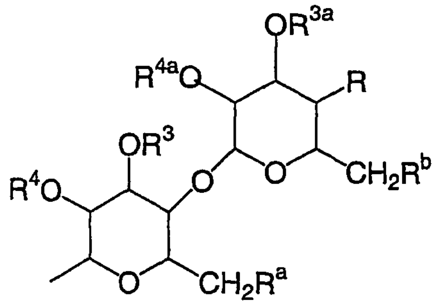 and 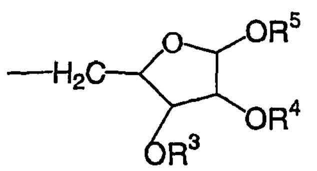 ,

(sugar derivatives)
wherein R, $R^a$ and $R^b$ are each independently selected from the group consisting of H, -OH, halo, -$NH_2$, azido, $(C_1\text{-}C_6)$alkoxy$(C_1\text{-}C_6)$alkoxy or -W-$R^{30}$;
W is independently selected from the group consisting of -NH-C(O)-, -O-C(O)-, -O-C(O)-N($R^{31}$)-, -NH-C(O)-N($R^{31}$)- and -O-C(S)-N($R^{31}$)-;
$R^2$ and $R^6$ are each independently selected from the group consisting of H,
$(C_1\text{-}C_6)$alkyl, acetyl, aryl and aryl$(C_1\text{-}C_6)$alkyl;
$R^3$, $R^4$, $R^5$, $R^7$, R3a and R4a are each independently selected from the group consisting of H, $(C_1\text{-}C_6)$alkyl, acetyl, aryl$(C_1\text{-}C_6)$alkyl, -C(O)$(C_1\text{-}C_6)$alkyl and -C(O)aryl;
$R^{30}$ is independently selected from the group consisting of $R^{32}$-substituted T, $R^{32}$-substituted-T-$(C_1\text{-}C_6)$alkyl, $R^{32}$-substituted-$(C_2\text{-}C_4)$alkenyl, $R^{32}$-substituted-$(C_1\text{-}C_6)$alkyl, $R^{32}$-substituted-$(C_3$-C7)cycloalkyl and $R^{32}$-substituted-$(C_3$-C7)cycloalkyl$(C_1\text{-}C_6)$alkyl;

$R^{31}$ is independently selected from the group consisting of H and $(C_1-C_4)$alkyl;
T is independently selected from the group consisting of phenyl, furyl, thienyl, pyrrolyl, oxazolyl, isoxazolyl, thiazolyl, isothiazolyl, benzothiazolyl, thiadiazolyl, pyrazolyl, imidazolyl and pyridyl;
$R^{32}$ is independently selected from 1-3 substituents which are each independently selected from the group consisting of H, halo, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, - $C(O)-N((C_1-C_4)alkyl)_2$, $-C(O)-(C_1-C_4)$ alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$ alkoxycarbonyl-substituted pyrrolidinyl, piperidinyl, N-methylpiperazinyl, indolinyl or morpholinyl group;
$G^1$ is represented by the structure:

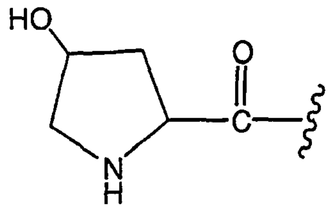
,
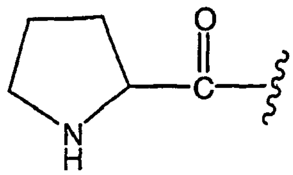
or
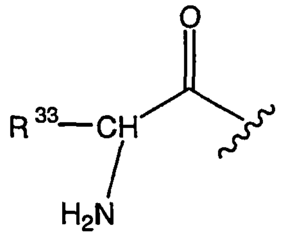

wherein $R^{33}$ is independently selected from the group consisting of unsubstituted alkyl, $R^{34}$-substituted alkyl, $(R^{35})(R^{36})$alkyl-,

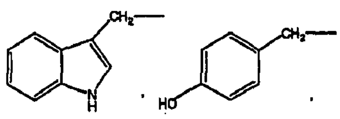
,
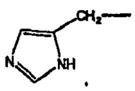
,
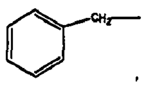
,

$R^{34}$ is one to three substituents, each $R^{34}$ being independently selected from the group consisting of HOOC-, HO-, HS-, $(CH_3)S-$, $H_2N-$, $(NH_2)(NH)C(NH)-$, $(NH_2)C(O)-$ and $HOOCCH(NH_3^+)CH_2SS-$;
$R^{35}$ is independently selected from the group consisting of H and $NH_2-$;
$R^{36}$ is independently selected from the group consisting of H, unsubstituted alkyl, $R^{34}$-substituted alkyl, unsubstituted cycloalkyl and $R^{34}$-substituted cycloalkyl;
$G^2$ is represented by the structure:

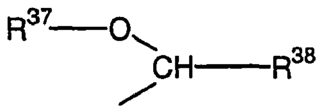

wherein $R^{37}$ and $R^{38}$ are each independently selected from the group consisting of $(C_1-C_6)$alkyl and aryl;
$R^{26}$ is one to five substituents, each $R^{26}$ being independently selected from the group consisting of:

a) H;
b) -OH;
c) $-OCH_3$;
d) fluorine;
e) chlorine;
f) -O-G;
g) $-O-G^1$;
h) $-O-G^2$;
i) $-SO_3H$; and
j) $-PO_3H$;

provided that when $R^1$ is H, $R^{26}$ is not H, -OH, $-OCH_3$ or -O-G;

$Ar^1$ is aryl, $R^{10}$-substituted aryl, heteroaryl or $R^{10}$-substituted heteroaryl;
$Ar^2$ is aryl, $R^{11}$-substituted aryl, heteroaryl or $R^{11}$-substituted heteroaryl;
L is selected from the group consisting of:

a) a covalent bond;
b) $-(CH_2)_q-$, wherein q is 1-6;
c) $-(CH_2)_e-E-(CH_2)_r-$, wherein E is -O-, -C(O)-, phenylene, $-NR^{22}$ - or $-S(O)_{0-2}-$, e is 0-5 and r is 0-5, provided that the sum of e and r is 1-6;
d) $-(C_2-C_6)$alkenylene-;
e) $-(CH_2)_f-V-(CH_2)_g-$, wherein V is $C_3$-$C_6$cycloalkylene, f is 1-5 and g is 0-5, provided that the sum of f and g is 1-6; and
f)

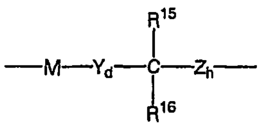 , 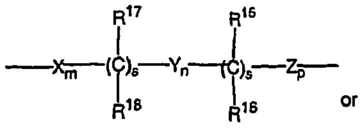 or 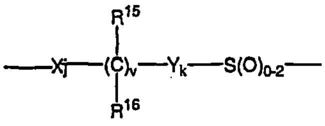 ;

wherein M is -O-, -S-, -S(O)- or $-S(O)_2-$;

X, Y and Z are each independently selected from the group consisting of $-CH_2-$, $-CH(C_1-C_6)$alkyl- and $-C(di-(C_1-C_6)$ alkyl)-;
$R^8$ is selected from the group consisting of H and alkyl;
$R^{10}$ and $R^{11}$ are each independently selected from the group consisting of 1-3 substituents which are each independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, $-CH=CH-COOR^{19}$, $-CF_3$, -CN, $-NO_2$ and halo;
$R^{15}$ and $R^{17}$ are each independently selected from the group consisting of $-OR^{19}$, $-OC(O)R^{19}$, $-OC(O)OR^{21}$, $-OC(O)NR^{19}R^{20}$;
$R^{16}$ and $R^{18}$ are each independently selected from the group consisting of H, $(C_1-C_6)$alkyl and aryl;
or $R^{15}$ and $R^{16}$ together are =O, or $R^{17}$ and $R^{18}$ together are =O;
d is 1, 2 or 3;
h is 0, 1, 2, 3 or 4;
s is 0 or 1;
t is 0 or 1;
m, n and p are each independently selected from 0-4;
provided that at least one of s and t is 1, and the sum of m, n, p, s and t is 1-6; provided that when p is 0 and t is 1, the sum of m, n and p is 1-5; and provided that when p is 0 and s is 1, the sum of m, t and n is 1-5;
v is 0 or 1;
j and k are each independently 1-5, provided that the sum of j, k and v is 1-5;
Q is a bond, $-(CH_2)_q-$, wherein q is 1-6, or, with the 3-position ring carbon of the azetidinone, forms the spiro group

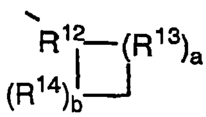 ;

wherein $R^{12}$ is

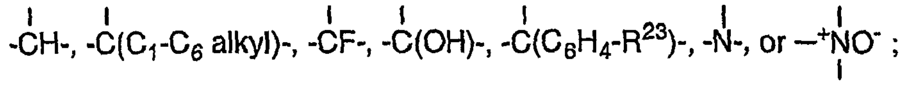

$R^{13}$ and $R^{14}$ are each independently selected from the group consisting of $-CH_2-$, $-CH(C_1-C_6\ alkyl)-$, $-C(di-(C_1-C_6)\ alkyl)$, -CH=CH- and $-C(C_1-C_6\ alkyl)=CH-$; or $R^{12}$ together with an adjacent $R^{13}$, or $R^{12}$ together with an adjacent $R^{14}$, form a -CH=CH- or a $-CH=C(C_1-C_6\ alkyl)-$ group;

a and b are each independently 0, 1, 2 or 3, provided both are not zero; provided that when $R^{13}$ is -CH=CH- or $-C(C_1-C_6\ alkyl)=CH-$, a is 1; provided that when $R^{14}$ is -CH=CH- or $-C(C_1-C_6\ alkyl)=CH-$, b is 1; provided that when a is 2 or 3, the $R^{13}$'s can be the same or different; and provided that when b is 2 or 3, the $R^{14}$'s can be the same or different; and when Q is a bond and L is

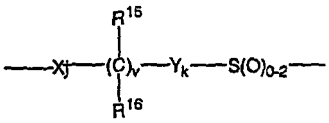

then $Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are each independently selected from the group consisting of H, $(C_1-C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

$R^{23}$ and $R^{24}$ are each independently selected from the group consisting of 1-3 substituents which are each independently selected from the group consisting of H, $(C_1-C_6)$alky), $(C_1-C_6)$alkoxy, -COOH, $NO_2$, $-NR^{19}R^{20}$, -OH and halo; and

R25 is H, -OH or $(C_1-C_6)$alkoxy.

Examples of compounds of Formula (IX) which are useful in the methods and combinations of the present invention and methods for making such compounds are disclosed in U.S. Patent Application Serial No. 10/166,942, filed June 11, 2002, incorporated herein by reference.

An example of a useful compound of this invention is one represented by the formula X:

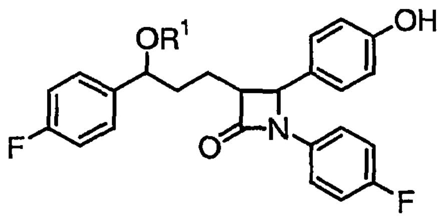

X

wherein $R^1$ is defined as above.

A more preferred compound is one represented by formula XI:

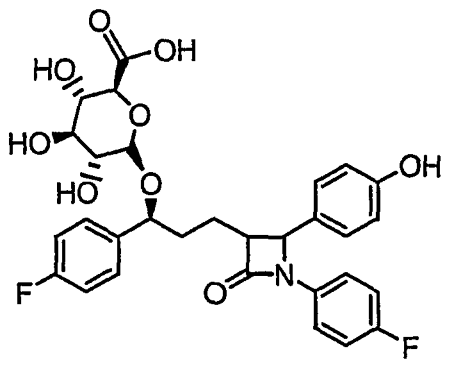

(XI).

Another useful compound is represented by Formula XII:

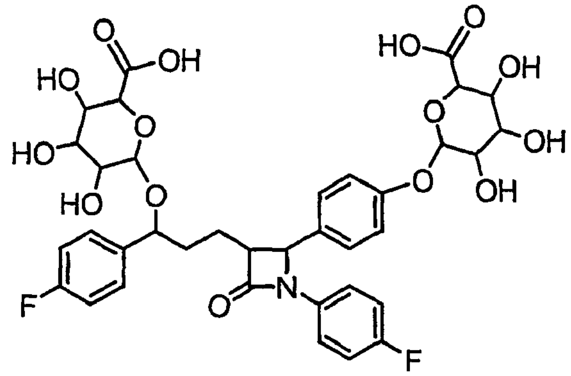

XII

Other useful substituted azetidinone compounds include N-sulfonyl-2-azetidinones such as are disclosed in U.S. Patent No. 4,983,597, ethyl 4-(2-oxoazetidin-4-yl)phenoxy-alkanoates such as are disclosed in Ram et al., Indian J. Chem. Sect. B. 29B, 12 (1990), p. 1134-7, and diphenyl azetidinones and derivatives disclosed in U.S. Patent Publication Nos. 2002/0039774, 2002/0128252, 2002/0128253 and 2002/0137689, and WO 2002/066464, each of which is incorporated by reference herein.

The compounds of Formulae I-XII can be prepared by known methods, including the methods discussed above and, for example, WO 93/02048 describes the preparation of compounds wherein -$R^1$-Q- is alkylene, alkenylene or alkylene interrupted by a hetero atom, phenylene or cycloalkylene; WO 94/17038 describes the preparation of compounds wherein Q is a spirocyclic group; WO 95/08532 describes the preparation of compounds wherein -$R^1$-Q- is a hydroxy-substituted alkylene group; PCT/US95/03196 describes compounds wherein -$R^1$-Q- is a hydroxy-substituted alkylene attached to the $Ar^1$ moiety through an -O- or $S(O)_{0-2}$- group; and U.S. Serial No. 08/463,619, filed June 5, 1995, describes the preparation of compounds wherein -$R^1$-Q- is a hydroxy-substituted alkylene group attached the azetidinone ring by a -$S(O)_{0-2}$- group.

The daily dose of the sterol absorption inhibitor(s) administered to the subject can range from about 0.1 to about 1000 mg per day, preferably about 0.25 to about 50 mg/day, and more preferably about 10 mg per day, given in a single dose or 2-4 divided doses. The exact dose, however, is determined by the attending clinician and is dependent on the potency of the compound administered, the age, weight, condition and response of the patient.

For administration of pharmaceutically acceptable salts of the above compounds, the weights indicated above refer to the weight of the acid equivalent or the base equivalent of the therapeutic compound derived from the salt.

In one embodiment of the present invention, the compositions or therapeutic combinations can further comprise one or more pharmacological or therapeutic agents or drugs such as cholesterol biosynthesis inhibitors and/or lipid-lowering agents discussed below.

In another embodiment, the composition or treatment can further comprise one or more cholesterol biosynthesis inhibitors coadministered with or in combination with the lipid modulating agent and substituted azetidinone or substituted β-tactam discussed above.

Non-limiting examples of cholesterol biosynthesis inhibitors for use in the compositions, therapeutic combinations and methods of the present invention include competitive inhibitors of HMG CoA reductase, the rate-limiting step in cholesterol biosynthesis, squalene synthase inhibitors, squalene epoxidase inhibitors and mixtures thereof. Non-limiting examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, CI-981 , rivastatin (sodium 7-(4-fluorophenyl)-2,6-diisopropyl-5-methoxymethylpyridin-3-yl)-3,5-dihydroxy-6-heptanoate), rosuvastatin, pitavastatin (such as NK-104 of Negma Kowa of Japan); HMG CoA synthetase inhibitors, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin, rosuvastatin and simvastatin. The most preferred HMG CoA reductase inhibitor is simvastatin. A preferred combination product containing ezetimibe and simvastatin that can be coadministered with the lipid modulating agent is VYTORIN™ ezetimibe/simvastatin that is commercially available from MSP Pharmaceuticals, Inc.

Generally, a total daily dosage of cholesterol biosynthesis inhibitor(s) can range from about 0.1 to about 160 mg per day, and preferably about 0.2 to about 80 mg/day in single or 2-3 divided doses.

In another preferred embodiment, the composition or treatment comprises the compound of Formula (II) in combination with one or more lipid modulating agent(s) and one or more cholesterol biosynthesis inhibitors. In this embodiment, preferably the lipid modulating agent is ETC-216. Preferably the cholesterol biosynthesis inhibitor comprises one or more HMG CoA reductase inhibitors, such as, for example, lovastatin, pravastatin and/or simvastatin. More preferably, the composition or treatment comprises the compound of Formula (II) in combination with simvastatin and ETC-216.

In another alternative embodiment, the compositions, therapeutic combinations or methods of the present invention can further comprise one or more bile acid sequestrants (insoluble anion exchange resins), coadministered with or in combination with the lipid modulating agent(s) and substituted azetidinone or substituted β-lactam discussed above.

Bile acid sequestrants bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of apo B/E (LDL) receptors that bind LDL from plasma to further reduce cholesterol levels in the blood.

Non-limiting examples of suitable bile acid sequestrants include cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quaternized polystyrenes, saponins and mixtures thereof. Suitable inorganic cholesterol sequestrants include bismuth salicylate plus montmorillonite clay, aluminum hydroxide and calcium carbonate antacids.

Generally, a total daily dosage of bile acid sequestrant(s) can range from about 1 to about 50 grams per day, and preferably about 2 to about 16 grams per day in single or 2-4 divided doses.

In an alternative embodiment, the compositions or treatments of the present invention can further comprise one or more ileal bile acid transport ("IBAT") inhibitors (or apical sodium co-dependent bile acid transport ("ASBT") inhibitors). The IBAT inhibitors can inhibit bile acid transport to reduce LDL cholesterol levels. Non-limiting examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727 which is incorporated herein by reference.

Generally, a total daily dosage of IBAT inhibitor(s) can range from about 0.01 to about 1000 mg/day, and preferably about 0.1 to about 50 mg/day in single or 2-4 divided doses.

In another alternative embodiment, the compositions or treatments of the present invention can further comprise nicotinic acid (niacin) and/or derivatives thereof.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from about 500 to about 10,000 mg/day, preferably about 1000 to about 8000 mg/day, and more preferably about 3000 to about 6000 mg/day in single or divided doses.

In another alternative embodiment, the compositions or treatments of the present invention can further comprise one or more AcylCoA:Cholesterol *O*-acyltransferase ("ACAT") Inhibitors, which can reduce LDL and VLDL levels. ACAT is an enzyme responsible for esterifying excess intracellular cholesterol and may reduce the synthesis of VLDL, which is a product of cholesterol esterification, and overproduction of apo B-100-containing lipoproteins.

Non-limiting examples of useful ACAT inhibitors include avasimibe ([[2,4,6-tris(1-methylethyl)phenyl]acetyl] sulfamic acid, 2,6-bis(1-methylethyl)phenyl ester, formerly known as CI-1011), HL-004, lecimibide (DuP-128) and CL-277082 (*N*-(2,4-difluorophenyl)-*N*-[[4-(2,2-dimethylpropyl)phenyl]methyl]-*N*-heptylurea). See P. Chang et al., "Current, New and Future Treatments in Dyslipidaemia and Atherosclerosis", Drugs 2000 Jul;60(1); 55-93, which is incorporated by reference herein.

Generally, a total daily dosage of ACAT inhibitor(s) can range from about 0.1 to about 1000 mg/day in single or 2-4 divided doses.

In another alternative embodiment, the compositions or treatments of the present invention can further comprise one or more Cholesteryl Ester Transfer Protein ("CETP") Inhibitors. CETP is responsible for the exchange or transfer of cholesteryl ester carrying HDL and triglycerides in VLDL.

Non-limiting examples of suitable CETP inhibitors are disclosed in PCT Patent Application No. WO 00/38721 and U.S. Patent No. 6,147,090, which are incorporated herein by reference. Pancreatic cholesteryl ester hydrolase (pCEH) inhibitors such as WAY-121898 also can be coadministered with or in combination.

Generally, a total daily dosage of CETP inhibitor(s) can range from about 0.01 to about 1000 mg/day, and preferably about 0.5 to about 20 mg/kg body weight/day in single or divided doses.

In another alternative embodiment, the compositions or treatments of the present invention can further comprise probucol or derivatives thereof (such as AGI-1067 and other derivatives disclosed in U.S. Patents Nos. 6,121,319 and 6,147,250), which can reduce LDL levels.

Generally, a total daily dosage of probucol or derivatives thereof can range from about 10 to about 2000 mg/day, and preferably about 500 to about 1500 mg/day in single or 2-4 divided doses.

In another alternative embodiment, the compositions or treatments of the present invention can further comprise low-density lipoprotein (LDL) receptor activators. Non-limiting examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from about 1 to about 1000 mg/day in single or 2-4 divided doses.

In another alternative embodiment, the compositions or treatments of the present invention can further comprise fish oil, which contains Omega 3 fatty acids (3-PUFA), which can reduce VLDL and triglyceride levels. Generally, a total daily dosage of fish oil or Omega 3 fatty acids can range from about 1 to about 30 grams per day in single or 2-4 divided doses.

In another alternative embodiment, the compositions or treatments of the present invention can further comprise natural water soluble fibers, such as psyllium, guar, oat and pectin, which can reduce cholesterol levels. Generally, a total daily dosage of natural water soluble fibers can range from about 0.1 to about 10 grams per day in single or 2-4 divided doses.

In another alternative embodiment, the compositions or treatments of the present invention can further comprise plant sterols, plant stanols and/or fatty acid esters of plant stanols, such as sitostanol ester used in BENECOL® margarine, which can reduce cholesterol levels. Generally, a total daily dosage of plant sterols, plant stanols and/or fatty acid esters of plant stanols can range from about 0.5 to about 20 grams per day in single or 2-4 divided doses.

In another alternative embodiment, the compositions or treatments of the present invention can further comprise antioxidants, such as probucol, tocopherol, ascorbic acid, β-carotene and selenium, or vitamins such as vitamin $B_6$ or vitamin $B_{12}$. Generally, a total daily dosage of antioxidants or vitamins can range from about 0.05 to about 10 grams per day in single or 2-4 divided doses.

In another alternative embodiment, the compositions or treatments of the present invention can further comprise monocyte and macrophage inhibitors such as polyunsaturated fatty acids (PUFA), thyroid hormones including throxine analogues such as CGS-26214 (a thyroxine compound with a fluorinated ring), gene therapy and use of recombinant proteins such as recombinant apo E. Generally, a total daily dosage of these agents can range from about 0.01 to about 1000 mg/day in single or 2-4 divided doses.

Also useful with the present invention are compositions or therapeutic combinations that further comprise hormone replacement agents and compositions. Useful hormone agents and compositions for hormone replacement therapy of the present invention include androgens, estrogens, progestins, their pharmaceutically acceptable salts and derivatives thereof. Combinations of these agents and compositions are also useful.

The dosage of androgen and estrogen combinations vary, desirably from about 1 mg to about 4 mg androgen and from about 1 mg to about 3 mg estrogen. Examples include, but are not limited to, androgen and estrogen combinations such as the combination of esterified estrogens (sodium estrone sulfate and sodium equilin sulfate) and methyltestosterone (17-hydroxy-17-methyl-, (17B)- androst-4-en-3-one) available from Solvay Pharmaceuticals, Inc., Marietta, GA, under the tradename Estratest.

Estrogens and estrogen combinations may vary in dosage from about 0.01 mg up to 8 mg, desirably from about 0.3 mg to about 3.0 mg. Examples of useful estrogens and estrogen combinations include:

(a) the blend of nine (9) synthetic estrogenic substances including sodium estrone sulfate, sodium equilin sulfate, sodium 17 α -dihydroequilin sulfate, sodium 17 α -estradiol sulfate, sodium 17 β -dihydroequilin sulfate, sodium 17 α -dihydroequilenin sulfate, sodium 17 β -dihydroequilenin sulfate, sodium equilenin sulfate and sodium 17 β -estradiol sulfate; available from Duramed Pharmaceuticals, Inc., Cincinnati, OH, under the tradename Cenestin;
(b) ethinyl estradiol (19-nor-17 α -pregna-1,3,5(10)-trien-20-yne-3,17-diol; available by Schering Plough Corporation, Kenilworth, NJ, under the tradename Estinyl;
(c) esterified estrogen combinations such as sodium estrone sulfate and sodium equilin sulfate; available from Solvay under the tradename Estratab and from Monarch Pharmaceuticals, Bristol, TN, under the tradename Menest;
(d) estropipate (piperazine estra-1,3,5(10)-trien-17-one, 3-(sulfooxy)-estrone sulfate); available from Pharmacia & Upjohn, Peapack, NJ, under the tradename Ogen and from Women First Health Care, Inc., San Diego, CA, under the tradename Ortho-Est; and
(e) conjugated estrogens (17 α-dihydroequilin, 17 α-estradiol, and 17 β-dihydroequilin); available from Wyeth-Ayerst Pharmaceuticals, Philadelphia, PA, under the tradename Premarin.

Progestins and estrogens may also be administered with a variety of dosages, generally from about 0.05 to about 2.0 mg progestin and about 0.001 mg to about 2 mg estrogen, desirably from about 0.1 mg to about 1 mg progestin and about 0.01 mg to about 0.5 mg estrogen. Examples of progestin and estrogen combinations that may vary in dosage and regimen include:

(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename Activella;
(b) the combination of levonorgestrel (d(-)-13 β-ethyl-17 α-ethinyl-17 β-hydroxygon- 4-en-3-one) and ethinyl estradial; available from Wyeth-Ayerst under the tradename Alesse, from Watson Laboratories, Inc., Corona, CA, under the tradenames Levora and Trivora, Monarch Pharmaceuticals, under the tradename Nordette, and from Wyeth-Ayerst under the tradename Triphasil;
(c) the combination of ethynodiol diacetate (19-nor-17 α-pregn-4-en-20-yne-3 β, 17-diol diacetate) and ethinyl estradiol; available from G.D. Searle & Co., Chicago, IL, under the tradename Demulen and from Watson under the tradename Zovia;
(d) the combination of desogestrel (13-ethyl-11-methylene-18,19-dinor-17 α-pregn- 4-en- 20-yn-17-ol) and ethinyl estradiol; available from Organon under the tradenames Desogen and Mircette, and from Ortho-McNeil Pharmaceutical, Raritan, NJ, under the tradename Ortho-Cept;
(e) the combination of norethindrone and ethinyl estradiol; available from Parke-Davis, Morris Plains, NJ, under the tradenames Estrostep and femhrt, from Watson under the tradenames Microgestin, Necon, and Tri-Norinyl, from Ortho-McNeil under the tradenames Modicon and Ortho-Novum, and from Warner Chilcott Laboratories, Rockaway, NJ, under the tradename Ovcon;
(f) the combination of norgestrel ( (±)-13-ethyl-17-hydroxy-18, 19-dinor-17 α-preg-4-en-20-yn-3-one) and ethinyl estradiol; available from Wyeth-Ayerst under the tradenames Ovral and Lo/Ovral, and from Watson under the tradenames Ogestrel and Low-Ogestrel;
(g) the combination of norethindrone, ethinyl estradiol, and mestranol (3-methoxy-19-nor-17 α-pregna-1,3,5(10)-trien-20-yn-17-ol); available from Watson under the tradenames Brevicon and Norinyl;
(h) the combination of 17 β-estradiol (estra-1,3,5(10)-triene-3,17 β-diol) and micronized norgestimate (17 α-17-(Acetyloxyl)-13-ethyl-18,19-dinorpregn-4-en-20-yn-3-one3-oxime); available from Ortho-McNeil under the tradename Ortho-Prefest;
(i) the combination of norgestimate (18,19-dinor-17-pregn-4-en-20-yn-3-one, 17--(acetyloxy)-13-ethyl-,oxime, (17 (α)-(+)-) and ethinyl estradiol; available from Ortho-McNeil under the tradenames Ortho Cyclen and Ortho Tri-Cyclen; and
(j) the combination of conjugated estrogens (sodium estrone sulfate and sodium equilin sulfate) and medroxyprogesterone acetate (20-dione, 17-(acetyloxy)-6-methyl-, (6(α))- pregn-4-ene-3); available from Wyeth-Ayerst under the tradenames Premphase and Prempro.

In general, a dosage of progestins may vary from about .05 mg to about 10 mg or up to about 200 mg if

microsized progesterone is administered. Examples of progestins include norethindrone; available from ESI Lederle, Inc., Philadelphia, PA, under the tradename Aygestin, from Ortho-McNeil under the tradename Micronor, and from Watson under the tradename Nor-QD; norgestrel; available from Wyeth-Ayerst under the tradename Ovrette; micronized progesterone (pregn-4-ene-3, 20-dione); available from Solvay under the tradename Prometrium; and medroxyprogesterone acetate; available from Pharmacia & Upjohn under the tradename Provera.

The compositions, therapeutic combinations or methods of the present invention can further comprise one or more obesity control medications. Useful obesity control medications include, but are not limited to, drugs that reduce energy intake or suppress appetite, drugs that increase energy expenditure and nutrient-partitioning agents. Suitable obesity control medications include, but are not limited to, noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); alpha-blocking agents; kainite or AMPA receptor antagonists; leptin-lipolysis stimulated receptors; phosphodiesterase enzyme inhibitors; compounds having nucleotide sequences of the mahogany gene; fibroblast growth factor-10 polypeptides; monoamine oxidase inhibitors (such as befloxatone, moclobemide, brofaromine, phenoxathine, esuprone, befol, toloxatone, pirlindol, amiflamine, sercloremine, bazinaprine, lazabemide, milacemide and caroxazone); compounds for increasing lipid metabolism (such as evodiamine compounds); and lipase inhibitors (such as orlistat). Generally, a total dosage of the above-described obesity control medications can range from 1 to 3,000 mg/day, desirably from about 1 to 1,000 mg/day and more desirably from about 1 to 200 mg/day in single or 2-4 divided doses.

The compositions, therapeutic combinations or methods of the present invention can further comprise one or more blood modifiers which are chemically different from the substituted azetidinone and substituted β-lactam compounds (such as compounds I-XII above) and the lipid modulating agents discussed above, for example, they contain one or more different atoms, have a different arrangement of atoms or a different number of one or more atoms than the sterol absorption inhibitor(s) or lipid modulating agents discussed above. Useful blood modifiers include but are not limited to anti-coagulants (argatroban, bivalirudin, dalteparin sodium, desirudin, dicumarol, lyapolate sodium, nafamostat mesylate, phenprocoumon, tinzaparin sodium, warfarin sodium); antithrombotic (anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox); fibrinogen receptor antagonists (roxifiban acetate, fradafiban, orbofiban, lotrafiban hydrochloride, tirofiban, xemilofiban, monoclonal antibody 7E3, sibrafiban); platelet inhibitors (cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole); platelet aggregation inhibitors (acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban); hemorrheologic agents (pentoxifylline); lipoprotein associated coagulation inhibitors; Factor VIIa inhibitors (4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides, naphthalene-2-sulfonic acid {1-[3-(aminoiminomethyl)-benzyl]-2-oxo-pyrrolidin-3-(S)-yl} amide trifluoroacetate, dibenzofuran-2-sulfonic acid {1-[3-(aminomethyl)-benzyl]-5-oxo-pyrrolidin-3-yl}-amide, tolulene-4-sulfonic acid {1-[3-(aminoiminomethyl)-benzyl]-2-oxo-pyrrolidin-3-(S)-yl}-amide trifluoroacetate, 3,4-dihydro-1H-isoquinoline-2-sulfonic acid {1-[3-(aminoiminomethyl)-benzyl]-2-oxo-pyrrolin-3-(S)-yl}-amide trifluoroacetate); Factor Xa inhibitors (disubstituted pyrazolines, disubstituted triazolines, substituted n-[(aminoiminomethyl)phenyl] propylamides, substituted n-[(aminomethyl)phenyl] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors).

The compositions, therapeutic combinations or methods of the present invention can further comprise one or more cardiovascular agents which are chemically different from the substituted azetidinone and substituted β-lactam compounds (such as compounds I-XI above) and the lipid modulating agents discussed above, for example, they contain one or more different atoms, have a different arrangement of atoms or a different number of one or more atoms than the sterol absorption inhibitor(s) or PPAR receptor activators discussed above. Useful cardiovascular agents include but are not limited to calcium channel blockers (clentiazem maleate, amlodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol

maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride); angiotensin II receptor antagonists (candesartan, irbesartan, losartan potassium, candesartan cilexetil, telmisartan); anti-anginal agents (amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions, therapeutic combinations or methods of the present invention can further comprise one or more antidiabetic medications for reducing blood glucose levels in a human. Useful antidiabetic medications include, but are not limited to, drugs that reduce energy intake or suppress appetite, drugs that increase energy expenditure and nutrient-partitioning agents. Suitable antidiabetic medications include, but are not limited to, sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Mixtures of any of the pharmacological or therapeutic agents described above can be used in the compositions and therapeutic combinations of the present invention.

The compositions and therapeutic combinations of the present invention can be administered to a subject or mammal in need of such treatment in a therapeutically effective amount to treat one or more conditions, for example vascular conditions such as atherosclerosis, hyperlipidaemia (including but not limited to hypercholesterolemia, hypertriglyceridaemia, sitosterolemia), vascular inflammation, stroke, diabetes, obesity, and/or reduce the level of sterol(s) in the plasma. The compositions and treatments can be administered by any suitable means which produce contact of these compounds with the site of action in the body, for example in the plasma, liver or small intestine of a mammal or human.

The pharmaceutical treatment compositions and therapeutic combinations of the present invention can further comprise one or more pharmaceutically acceptable carriers, one or more excipients and/or one or more additives. Non-limiting examples of pharmaceutically acceptable carriers include solids and/or liquids such as ethanol, glycerol, water and the like. The amount of carrier in the treatment composition can range from about 5 to about 99 weight percent of the total weight of the treatment composition or therapeutic combination. Non-limiting examples of suitable pharmaceutically acceptable excipients and additives include non-toxic compatible fillers, binders such as starch, disintegrants, buffers, preservatives, anti-oxidants, lubricants, flavorings, thickeners, coloring agents, emulsifiers and the like. The amount of excipient or additive can range from about 0.1 to about 90 weight percent of the total weight of the treatment composition or therapeutic combination. One skilled in the art would understand that the amount of carrier(s), excipients and additives (if present) can vary.

The treatment compositions of the present invention can be administered in any conventional dosage form, preferably an oral dosage form such as a capsule, tablet, powder, cachet, suspension or solution. The formulations and pharmaceutical compositions can be prepared using conventional pharmaceutically acceptable and conventional techniques. Several examples of preparation of dosage formulations are provided below.

The following formulations exemplify some of the dosage forms of this invention. In each formulation, the term "Active Compound I" designates a substituted azetidinone compound, β-lactam compound or any of the compounds of Formulae I-XI described herein above, or pharmaceutically acceptable salts or solvates thereof, and the term "Active Compound II" designates a lipid modulating agent described herein above.

EXAMPLE

Tablets

| No. | Ingredient | mg/tablet |
|---|---|---|
| 1 | Active Compound I | 10 |
| 2 | Lactose monohydrate NF | 55 |
| 3 | Microcrystalline cellulose NF | 20 |
| 4 | Povidone (K29-32) USP | 4 |
| 5 | Croscarmellose sodium NF | 8 |
| 6 | Sodium lauryl sulfate | 2 |
| 7 | Magnesium stearate NF | 1 |
| | Total | 100 |

In the present invention, the above-described tablet can be coadministered with a treatment comprising a dosage of Active Compound II, for example an infusion of ETC -216.

Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

It is contemplated that where the two active ingredients are administered as a single composition, the dosage forms disclosed above for substituted azetidinone or β-lactam compounds may readily be modified using the knowledge of one skilled in the art.

Since the present invention relates to treating conditions as discussed above, such as reducing the plasma sterol (especially cholesterol) concentrations or levels by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least one peroxisome proliferator-activated receptor activator and a separate pharmaceutical composition comprising at least one sterol absorption inhibitor as described above. The kit will preferably include directions for the administration of the separate components. The kit form is particularly advantageous when the separate components must be administered in different dosage forms (e.g., oral and parenteral) or are administered at different dosage intervals.

The treatment compositions and therapeutic combinations of the present invention can inhibit the intestinal absorption of cholesterol in mammals, as shown in the Example below, and can be useful in the treatment and/or prevention of conditions, for example vascular conditions, such as atherosclerosis, hypercholesterolemia and sitosterolemia, stroke, obesity and lowering of plasma levels of cholesterol in mammals, in particular in mammals.

In another embodiment of the present invention, the compositions and therapeutic combinations of the present invention can inhibit sterol or 5α-stanol absorption or reduce plasma concentration of at least one sterol selected from the group consisting of phytosterols (such as sitosterol, campesterol, stigmasterol and avenosterol) and/or 5α-stanol (such as cholestanol, 5α-campestanol, 5α-sitostanol), cholesterol and mixtures thereof. The plasma concentration can be reduced by administering to a mammal in need of such treatment an effective amount of at least one treatment composition or therapeutic combination comprising at least one lipid modulating agent and at least one sterol absorption inhibitor described above. The reduction in plasma concentration of sterols or 5α-stanols can range from about 1 to about 70 percent, and preferably about 10 to about 50 percent. Methods of measuring serum total blood cholesterol and total LDL cholesterol are well known to those skilled in the art and for example include those disclosed in PCT WO 99/38498 at page 11, incorporated by reference herein. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999), incorporated by reference herein.

The treatments of the present invention can also reduce the size or presence of plaque deposits in vascular vessels. The plaque volume can be measured using (IVUS), in which a tiny ultrasound probe is inserted into an artery to directly image and measure the size of atherosclerotic plaques, in a manner well know to those skilled in the art.

Illustrating the invention are the following examples that, however, are not to be considered as limiting the

invention to their details. Unless otherwise indicated, all parts and percentages in the following examples, as well as throughout the specification, are by weight.

**EXAMPLES**

PREPARATION OF COMPOUND OF FORMULA (II)

Step 1): To a solution of (S)-4-phenyl-2-oxazolidinone (41 g, 0.25 mol) in $CH_2Cl_2$ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g, 0.3 mol) was added as a solution in $CH_2Cl_2$ (375 ml) dropwise over 1 h, and the reaction was allowed to warm to 22°C. After 17 h, water and $H_2SO_4$ (2N, 100 ml), was added the layers were separated, and the organic layer was washed sequentially with NaOH (10%), NaCl (sat'd) and water. The organic layer was dried over $MgSO_4$ and concentrated to obtain a semicrystalline product.

Step 2): To a solution of $TiCl_4$ (18.2 ml, 0.165 mol) in $CH_2Cl_2$ (600 ml) at 0°C, was added titanium isopropoxide (16.5 ml, 0.055 mol). After 15 min, the product of Step 1 (49.0 g, 0.17 mol) was added as a solution in $CH_2Cl_2$ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in $CH_2Cl_2$ dropwise over 15 min, the reaction mixture was allowed to warm to 0°C, and $H_2SO_4$ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis (trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, $CH_3OH$ (10 ml), was added. The reaction mixture was washed with HCl (1 N), $NaHCO_3$ (1 N) and NaCl (sat'd.), and the organic layer was dried over $MgSO_4$.

Step 4): To a solution of the product of Step 3 (0.94 g, 2.2 mmol) in $CH_3OH$ (3 ml), was added water (1 ml) and $LiOH{\cdot}H_2O$ (102 mg, 2.4 mmole). The reaction mixture was stirred at 22ºC for 1 h and then additional $LiOH{\cdot}H_2O$ (54 mg, 1.3 mmole) was added. After a total of 2 h, HCl (1 N) and EtOAc was added, the layers were separated, the organic layer was dried and concentrated in *vacuo.* To a solution of the resultant product (0.91 g, 2.2 mmol) in $CH_2Cl_2$ at 22°C, was added ClCOCOCl (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1 M in THF, 4.4 ml, 4.4 mmol) and $ZnCl_2$ (0.6 g, 4.4 mmol) at 4°C, was added tetrakis (triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1 N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:

HRMS calc'd for $C_{24}H_{19}F_2NO_3$ = 408.1429, found 408.1411.

Step 6): To the product of Step 5 (0.95 g, 1.91 mmol) in THF (3 ml), was added (R)-tetrahydro-1-methyl-3,3-diphenyl-1H,3H-pyrrolo-[1,2-c][1,3,2] oxazaborole (120 mg, 0.43 mmol) and the mixture was cooled to -20°C. After 5 min, borohydride-dimethylsulfide complex (2M in THF, 0.85 ml, 1.7 mmol) was added dropwise over 0.5 h. After a total of 1.5 h , $CH_3OH$ was added followed by HCl (1 N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. $^1H$ in $CDCl_3$ d H3 = 4.68. J = 2.3 Hz. Cl ($M^+H$) 500.

Use of (S)-tetra-hydro-1-methyl-3,3-dipheny)-1H,3H-pyrrolo-[1,2-c][1,3,2] oxazaborole gives the corresponding 3 (R)-hydroxypropyl azetidinone (compound 6B-1). $^1H$ in $CDCl_3$ d H3 = 4.69. J = 2.3 Hz. Cl ($M^+H$) 500.

To a solution of compound 6A-1 (0.4 g, 0.8 mmol) in ethanol (2 ml), was added 10% Pd/C (0.03 g) and the reaction mixture was stirred under a pressure (60 psi) of $H_2$ gas for 16 h. The reaction mixture was filtered and the solvent was concentrated to obtain compound 6A. Mp 164-166°C; Cl ($M^+H$) 410.

$[\alpha]_D^{25} = -28.1^\circ$ (c 3, $CH_3OH$) . Elemental analysis calc'd for $C_{24}H_{21}F_2NO_3$: C 70.41; H 5.17; N 3.42; found C 70.25; H 5.19; N 3.54.

Similarly treat compound 6B-1 to obtain compound 6B.

Mp 129.5-132.5°C; CI ($M^+H$) 410. Elemental analysis calc'd for $C_{24}H_{21}F_2NO_3$: C 70.41; H 5.17; N 3.42; found C 70.30; H 5.14; N 3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of $H_2$ gas for 16 h. The reaction mixture was filtered and the solvent was concentrated to afford a 1:1 mixture of compounds 6A and 6B.

It will be appreciated by those skilled in the art that changes could be made to the embodiments described above without departing from the broad inventive concept thereof. It is understood, therefore, that this invention is not limited to the particular embodiments disclosed, but it is intended to cover modifications which are within the spirit and scope of the invention, as defined by the appended claims.

## Claims

**1.** A composition comprising:

(a) at least one lipid modulating agent; and
(b) at least one substituted azetidinone compound or substituted β-lactam compound or salt or solvate thereof.

**2.** The composition according to claim 1, wherein the substituted azetidinone compound is represented by Formula (I):

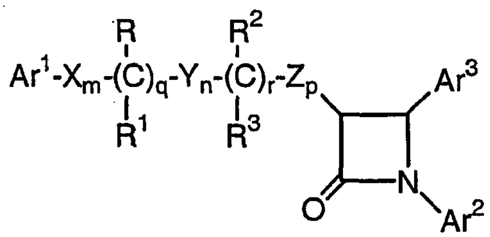

(I)

or pharmaceutically acceptable salts or solvates thereof, wherein in Formula (I) above:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene) $COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

3. The composition according to claim 1, wherein the substituted azetidinone compound is represented by Formula (II) below:

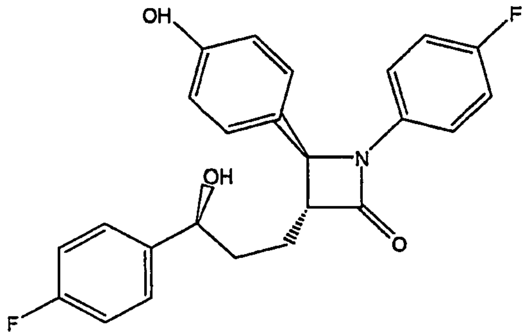

(II)

or pharmaceutically acceptable salt or solvate thereof.

4. The composition according to claim 1, wherein the lipid modulating agent is selected from the group consisting of synthetic HDL, phospholipids, phospholipids in combination with HDL associated and biologically active peptides derived therefrom, reverse lipid transport (RLT) peptides, enzymes associated with HDL, and apo E, alone or formulated in combination with liposomes or emulsions.

5. The composition according to claim 1, wherein the lipid modulating agent is a synthetic HDL complex comprising recombinant apolipoprotein A-I Milano and 1-palmitoyl-2-oleoyl phosphatidyl choline complex.

6. A composition comprising: (a) at least one lipid modulating agent; and (b) a compound represented by Formula (II) below:

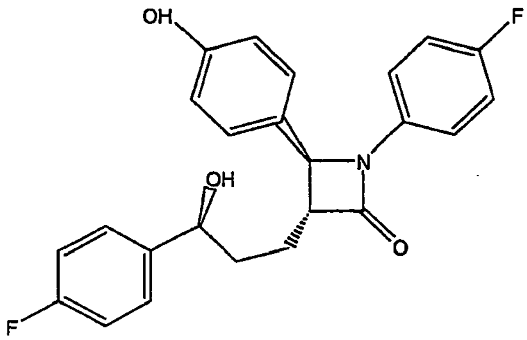

(II)

or pharmaceutically acceptable salt or solvate thereof.

7. A therapeutic combination comprising: (a) a first amount of at least one lipid modulating agent; and (b) a second amount of at least one substituted azetidinone compound or substituted β-lactam compound or pharmaceutically acceptable salt or solvate thereof, wherein the first amount and the second amount together comprise a therapeutically effective amount for the treatment or prevention of a vascular condition, diabetes, obesity or lowering a concentration of a sterol in plasma of a subject.

8. A pharmaceutical composition for the treatment or prevention of a vascular condition, diabetes, obesity or lowering a concentration of a sterol in plasma of a subject, comprising a therapeutically effective amount of a composition or therapeutic combination of any of claims 1, 6 or 7 and a pharmaceutically acceptable carrier.

9. A method of treating or preventing a vascular condition, diabetes, obesity or lowering a concentration of a sterol in plasma of a subject, comprising the step of administering to a mammal in need of such treatment an effective amount of a composition or therapeutic combination of any of claims 1, 6, 7 or 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5767115 A
- US 5624920 A
- US 5668990 A
- US 5656624 A
- US 5688787 A
- US 5756470 A
- US 20020137690 A
- US 20020137689 A
- WO 2002066464 A
- US 5846966 A
- US 5661145 A
- US 20030109442 A
- WO 9523592 A
- US 4868116 A
- US 4980286 A
- WO 9002806 A
- WO 8907136 A
- US 5631365 A
- US 6207822 B
- WO 02079174 A
- WO 9302048 A
- US 5688990 A
- US 5698548 A
- US 16694202 A
- US 4983597 A
- US 20020039774 A
- US 20020128252 A
- US 20020128253 A
- WO 9417038 A
- WO 9508532 A
- US 9503196 W
- US 08463619 B
- WO 0038727 A
- WO 0038721 A
- US 6147090 A
- US 6121319 A
- US 6147250 A
- WO 9938498 A


### Non-patent literature cited in the description


- **BREWER et al.** *Biochem. Biophys. Res. Commun,* 1978, vol. 80, 623-630
- **SEGREST et al.** *FEBS Lett,* 1974, vol. 38, 247-253
- **WEISGRABER et al.** *J. Clin. Invest,* 1980, vol. 66, 901-907
- **FRANCESCHINI et al.** *J. Clin. Invest,* 1980, vol. 66, 892-900
- **MIYAZAKI et al.** *Arterioscler Thromb Vasc Biol,* 1995, vol. 15, 1882-1888
- **BADIMON et al.** *Lab Invest,* 1989, vol. 60, 455-461
- *J Clin Invest,* 1990, vol. 85, 1234-1241
- **NANJEE et al.** *Arterioscier Thromb Vasc Biol,* 1999, vol. 19, 979-989
- **ERIKSSON et al.** *Circulation,* 1999, vol. 100, 594-598
- **BIELICKI ; ODA.** *Biochemistry,* 2002, vol. 41, 2089-2096
- **FRANCIS et al.** *Am. J. Pharmacogenomics,* 2001, vol. 1 (1), 55-66
- **TEIGER et al.** *J. Cardiovasc. Pharmacol,* 1999, vol. 33 (5), 726-732
- **WOLFF et al.** *Biotechniques,* 1991, vol. 11, 474-85
- **RAM et al.** *Cancer Res.,* 1993, vol. 53, 83-88
- **WOLFF, J. A. et al.** *Science,* 1990, vol. 247, 1465-1468
- **WOLFF, J. A.** *Nature,* 1991, vol. 352, 815-818
- Retroviral vectors for gene transfer. **VERMA, I. M.** MICROBIOLOGY. American Society for Microbiology, 1985, 229-232
- **MULLIGAN.** *Science,* 1993, vol. 260, 926-932
- **BERKNER et al.** *J. Virology,* 1987, vol. 61, 1213-1220
- **MASSIE et al.** *Mol. Cell. Biol,* 1986, vol. 6, 2872-2883
- **HAJ-AHMAD et al.** *J. Virology,* 1986, vol. 57, 267-274
- **DAVIDSON et al.** *J. Virology,* 1987, vol. 61, 1226-1239
- **ZHANG.** Generation and identification of recombinant adenovirus by liposome-mediated transfection and PCR analysis. *BioTechniques,* 1993, vol. 15, 868-872
- **MORSY.** *J. Clin. Invest.,* 1993, vol. 92, 1580-1586
- **KIRSHENBAUM.** *J. Clin. Invest,* 1993, vol. 92, 381-387
- **ROESSLER.** *J. Clin. Invest.,* 1993, vol. 92, 1085-1092

- **MOULLIER.** *Nature Genetics,* 1993, vol. 4, 154-159
- **LA SALLE.** *Science,* 1993, vol. 259, 988-990
- **GOMEZ-FOIX.** *J. Biol. Chem.,* 1992, vol. 267, 25129-25134
- **RICH.** *Human Gene Therapy,* 1993, vol. 4, 461-476
- **ZABNER.** *Nature Genetics,* 1994, vol. 6, 75-83
- **GUZMAN.** *Circulation Research,* 1993, vol. 73, 1201-1207
- **BOUT.** *Human Gene Therapy,* 1994, vol. 5, 3-10
- **ZABNER.** *Cell,* 1993, vol. 75, 207-216
- **CAILLAUD.** *Eur. J. Neuroscience,* 1993, vol. 5, 1287-1291
- **RAGOT.** *J. Gen. Virology,* 1993, vol. 74, 501-507
- **CHARDONNET ; DALES.** *Virology,* 1970, vol. 40, 462-477
- **BROWN ; BURLINGHAM.** *J. Virology,* 1973, vol. 12, 386-396
- **SVENSSON ; PERSSON.** *J. Virology,* 1985, vol. 55, 442-449
- **SETH et al.** *J. Virol,* 1984, vol. 51, 650-655
- **SETH et al.** *Mol. Cell. Biol,* 1984, vol. 4, 1528-1533
- **VARGA et al.** *J. Virology,* 1991, vol. 65, 6061-6070
- **WICKHAM et al.** *Cell,* 1993, vol. 73, 309-319
- **FIERS et al.** *Nature,* 1978, vol. 273, 113
- **GREENWAY, P. J. et al.** *Gene,* 1982, vol. 18, 355-360
- **LAIMINS, L. et al.** *Proc. Natl. Acad. Sci.,* 1981, vol. 78, 993
- **LUSKY, M. L. et al.** *Mol. Cell Bio,* 1983, vol. 3, 1108
- **BANERJI, J. L. et al.** *Cell,* 1983, vol. 33, 729
- **OSBORNE, T. F. et al.** *Mol. Cell Bio,* 1984, vol. 4, 1293
- **CHEN et al.** *Jpn. J. Pharmacol.,* 2002, vol. 89 (4), 327-336
- **KIPSHIDZE et al.** *J. Am. Coll. Cardio,* 2002, vol. 39 (10), 1686-1691
- **TURUNEN et al.** *Mol Ther,* 2002, vol. 6 (3), 306
- **RAM et al.** *Indian J. Chem. Sect. B.,* 1990, vol. 29B (12), 1134-7
- **P. CHANG et al.** Current, New and Future Treatments in Dyslipidaemia and Atherosclerosis. *Drugs,* July 2000, vol. 60 (1), 55-93
- **M. HUETTINGER et al.** Hypolipidemic activity of HOE-402 is Mediated by Stimulation of the LDL Receptor Pathway. *Arterioscler. Thromb,* 1993, vol. 13, 1005-12
- **H. GYLLING et al.** Serum Sterols During Stanol Ester Feeding in a Mildly Hypercholesterolemic Population. *J. Lipid Res,* 1999, vol. 40, 593-600